(19) Europäisches Patentamt
European Patent Office
Office européen des brevets

(11) Publication number: **0 259 977 A2**

(12) **EUROPEAN PATENT APPLICATION**

(21) Application number: 87307080.9

(22) Date of filing: 10.08.87

(51) Int. Cl.³: C 07 D 265/30
C 07 D 295/02, A 01 N 43/84
A 01 N 43/40, C 07 F 7/10
A 01 N 55/00

(30) Priority: 10.09.86 GB 8621790

(43) Date of publication of application:
16.03.88 Bulletin 88/11

(84) Designated Contracting States:
AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Anthony, Vivienne Margaret
4 The Croft
Maidenhead Berkshire(GB)

(72) Inventor: Urch, Christopher John
40 Dalcross Crown Wood
Bracknell Berkshire(GB)

(72) Inventor: Elliott, Alison Clare
14 Huntington Close Lower Earley
Nr. Reading Berkshire(GB)

(74) Representative: Alner, Henry Giveen Hamilton et al,
Imperial Chemical Industries PLC Legal Department:
Patents P.O. Box 6 Bessemer Road
Welwyn Garden City Herts, AL7 1HD(GB)

(54) **Tertiary amine compounds.**

(57) Compounds having plant fungicidal properties are of formula:

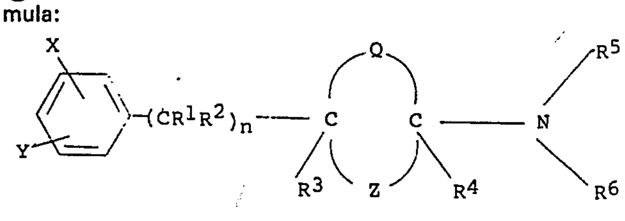

(I)

and stereoisomers thereof, wherein $R^1$, $R^2$, $R^3$ and $R^4$ each represents a hydrogen atom, a halogen atom, or an alkyl group containing from 1 to 4 carbon atoms but $R^4$ is not halogen, $R^5$ and $R^6$ each represents an alkyl group containing from 1 to 4 carbon atoms or $R^5$ and $R^6$ together with the adjacent nitrogen atom form a heterocyclic ring which may optionally contain an additional heteroatom, X and Y each represent a hydrogen atom, a halogen atom, or an alkyl, cycloalkyl, cycloalkylalkyl, alkenyl, alkynyl, aryl, aralkyl, alkoxy or aryloxy group, or a

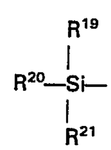

group wherein $R^{19}$, $R^{20}$ and $R^{21}$ can be an alkyl, alkenyl, alkynyl, cycloalkyl, cycloalkylalkyl, aryl or aralkyl group, n has the value 0 or 1 and Q and Z are bridging groups as defined; and acid addition salts and N-oxides thereof.

EP 0 259 977 A2

TERTIARY AMINE COMPOUNDS

This invention relates to tertiary amine compounds containing a cyclobutane, cyclopentane or cyclohexane ring which are useful as fungicides, to processes for preparing the compounds, to fungicidal compositions containing them, and to methods of using them to combat fungi, especially fungal infections in plants.

According to the invention there are provided compounds having the general formula (I) :

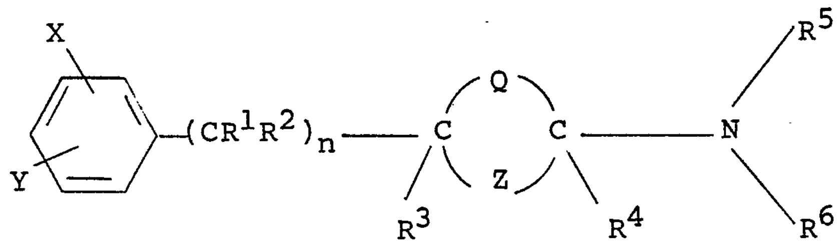

(I)

and stereoisomers thereof, wherein $R^1$, $R^2$ and $R^3$ each represents a hydrogen atom, a halogen atom, or an alkyl group containing from 1 to 4 carbon atoms, $R^4$ represents a hydrogen atom or an alkyl group containing from 1 to 4 carbon atoms, $R^5$ and $R^6$ each represents an alkyl group containing from 1 to 4 carbon atoms or $R^5$ and $R^6$ together with the adjacent nitrogen atom form a heterocyclic ring which may optionally contain an additional heteroatom, X and Y each represents a hydrogen atom, a halogen atom, or an alkyl, cycloalkyl, cycloalkylalkyl, alkenyl, alkynyl, aryl, aralkyl, alkoxy or aryloxy group, or a

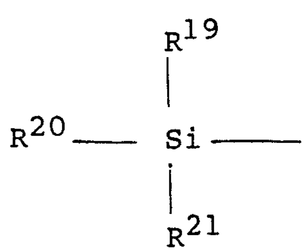

group wherein $R^{19}$, $R^{20}$ and $R^{21}$ can be an alkyl, alkenyl, alkynyl, cycloalkyl, cycloalkylalkyl, aryl or aralkyl group, n has the value 0 or 1 and Q is a bridging group having one of the following values $Q_1$, $Q_2$, $Q_3$ and $Q_4$ :

$Q_1$=

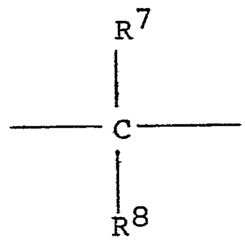

$Q_2$=

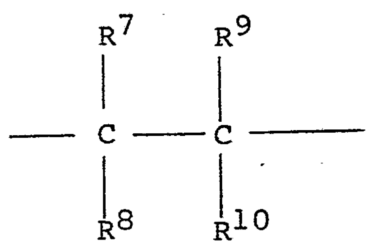

$Q_3$=

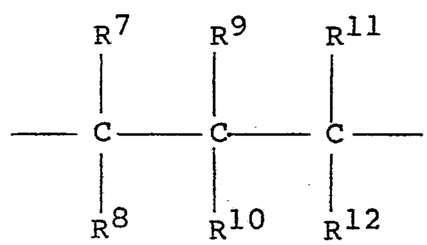

$Q_4$=

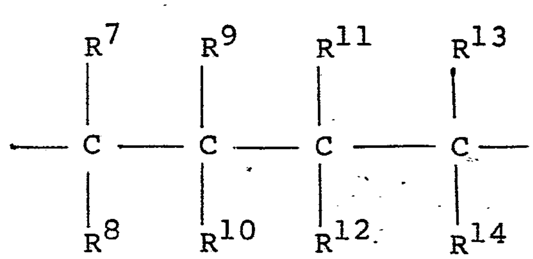

wherein $R^7$ to $R^{14}$ each independently represents a hydrogen atom, a halogen atom, a hydroxyl group or an alkyl group containing from 1 to 4 carbon atoms; Z is a bridging group which is a covalent linkage, denoted by $Z_o$, or has one of the following values $Z_1$ and $Z_2$ :

$Z_1=$ 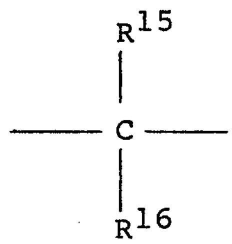

$Z_2=$ 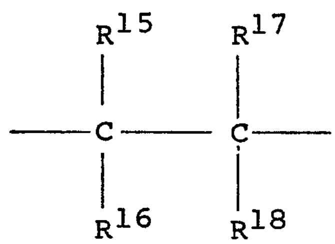

wherein $R^{15}$ to $R^{18}$ each independently has the same significance as $R^7$ to $R^{14}$ above; provided that

(i) when Z has the value $Z_o$, Q does not have the value $Q_1$;

(ii) the total number of carbon atoms forming part of the ring substance in formula (I) and contributed by the groups Q and Z does not exceed 4; and acid addition salts and N-oxides thereof.

The compounds of the invention are generally obtained in the form of mixtures of geometric isomers. However, these and other mixtures of optical isomers can be

separated into individual isomers by methods in the art and such isomers constitute a part of the present invention.

When $R^5$ and $R^6$, together with the adjacent N-atom, represent a heterocyclic ring this may be, for example, a piperidine, morpholine, thiomorpholine, pyrrolidine or piperazine ring and any of these rings may bear substituents such as one or more $C_{1-4}$ alkyl groups or a phenyl group, or a hydroxy-$C_{1-4}$-alkyl group.

Alkyl groups containing from 1 to 4 carbon atoms represented by $R^1$, $R^2$, $R^3$, $R^4$, $R^5$, $R^6$ and $R^7$ to $R^{18}$ may be either straight or branched chain groups, for example methyl, ethyl, n-propyl, iso-propyl, n-butyl, sec-butyl, iso-butyl, tert-butyl. Alkyl and alkoxy groups represented by X and Y, and $R^{19}$ to $R^{21}$ when alkyl, may be either straight or branched chain groups containing from 1 to 6 carbon atoms; cycloalkyl groups represented by X and Y and $R^{19}$ to $R^{21}$ may be $C_{3-6}$ cycloalkyl groups, e.g. cyclopropyl, cyclobutyl, cyclopentyl or cyclohexyl groups; or cycloalkylalkyl groups, e.g. $C_{4-9}$ cycloalkylalkyl groups.

Halogen atoms represented by $R^1$ to $R^3$, $R^7$ to $R^{18}$ and X and Y may be fluorine, chlorine or bromine atoms.

Alkenyl and alkynyl groups represented by X and Y may contain from 2 to 6 carbon atoms and for $R^{19}$ to $R^{21}$ from 2 to 4 carbon atoms.

Examples of X and Y when these are aryl, aralkyl, aryloxy or aralkoxy groups are phenyl, benzyl, phenoxy and benzyloxy. Examples of $R^{19}$ to $R^{21}$ when these are aryl and aralkyl are phenyl and benzyl. These rings may be substituted with halogen (e.g. fluorine, chlorine, or bromine), $C_{1-6}$ alkyl [e.g. methyl, ethyl, propyl (n- or iso-propyl) and butyl (n-, sec-, iso, or tert-butyl)], $C_{1-6}$ alkoxy (e.g. methoxy, ethoxy, propoxy and butoxy), halo-$C_{1-6}$-alkoxy (e.g. tri-fluoromethoxy), halo-$C_{1-6}$-alkyl (e.g. trifluoromethyl), nitro, phenyl and phenoxy.

Examples of X and Y when these are a silyl group are trimethylsily, triethylsilyl, t-butyldimethylsilyl, phenyldimethylsilyl and allyldimethylsilyl. The phenyl ring may thus be unsubstituted or substituted with ring substituents as defined above. The X and Y substituents may be at the 2-, 3- or 4-positions of the phenyl ring and the 3- and the 4-positions are preferred. The 4-position is preferred over the 3-position.

According to one particular aspect of the invention, there are provided compounds containing a cyclobutane ring and having the general formula (I) :

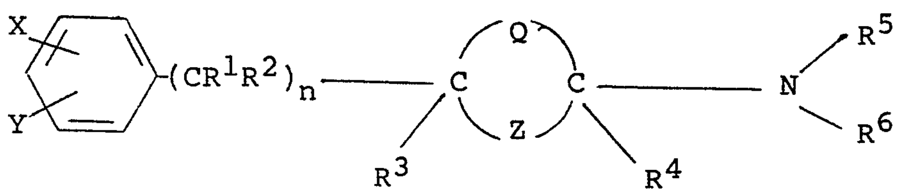

(I)

and stereoisomers and addition salts thereof, wherein either Q has the value $Q_2$ and Z has the value $Z_0$ as previously defined, or Q has the value $Q_1$ and Z has the value $Z_1$ as previously defined, the remaining symbols having the same significance as before. These two alternatives correspond to compounds in which the molecular residues :

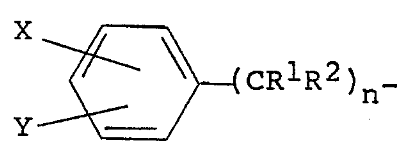

and

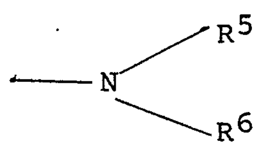

are attached to the cyclobutane ring in the 1,2- and 1,3-positions respectively. The 1,3-substituted compounds are preferred.

According to another particular aspect of the invention, there are provided compounds containing a cyclopentane ring and having the general formula (I) above, wherein Q has the value $Q_3$ and Z has the value $Z_0$, or Q has the value $Q_2$ and Z has the value $Z_1$, as previously defined, the remaining symbols having the same significance as before. These two possibilities correspond to substitution of the cyclopentane ring by the molecular residues referred to above in the 1,2- and 1,3-positions respectively. The 1,3-substituted compounds are preferred.

According to a further particular aspect of the invention, there are provided compounds containing a cyclohexane ring and having the general formula (I) above, wherein Q has the value $Q_4$ and Z has the value $Z_o$, or Q has the value $Q_3$ and Z has the value $Z_1$, or Q has the value $Q_2$ and Z has the value $Z_2$ as previously defined, the remaining symbols having the same significance as before. These three possibilities correspond to 1,2-, 1,3- or 1,4-substitution respectively of the cyclohexane ring by the two molecular residues referred to previously. The 1,3- and 1,4-substituted compounds are preferred.

Examples of the compounds of the invention are shown in Table I. These correspond to general formula I, the various substituents being shown in the columns.

In Table I the significance of the abbreviations is as follows :

| | | |
|---|---|---|
| _t_-Bu | represents | tertiary butyl |
| _s_-Bu | " | secondary butyl |
| _i_-Bu | " | isobutyl |
| _n_-Bu | " | normal butyl |
| _i_-Pr | " | isopropyl |
| TMS | " | trimethylsilyl |
| Me | " | methyl |
| Et | " | ethyl |
| Ph | " | phenyl |

TABLE I

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 1 | 4-t-Bu | H | - | H | H | $CH_2$ | $CH_2$ | 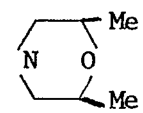 | Oil | cis Ar/N | $^1H$ NMR ($CDCl_3$, 270 MHz); 7.31 (2H, m), 7.19 (2H, m), 3.69 (2H, m), 3.11 (1H, tt, J=10.7 and 7.4 Hz), 2.72 (2H, d, J=10.7 Hz), 2.67 (1H, tt, J=9.4 and 6.7 Hz), 2.46 (2H, m), 1.99 (2H, m), 1.59 (2H, t, J=10.7 Hz), 1.31 (9H, s) and 1.17 (6H, d, J=6.0 Hz). |
| 2 | 4-t-Bu | H | - | H | H | $CH_2$ | $CH_2$ | 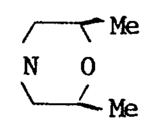 | Oil | trans Ar/N | $^1H$ NMR ($CDCl_3$, 270 MHz); 7.34 (2H, m), 7.22 (2H, m), 3.71 (2H, m), 3.47 (1H, sept., J=5.3 Hz), 2.91 (1H, pent, J=8.0 Hz), 2.79 (2H, d, J=10.7 Hz), 2.43 (2H, m), 2.34 (2H, m), 1.53 (2H, t, J=10.7 Hz), 1.32 (9H, s) and 1.18 (6H, d, J=6.7 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 3 | 4-t-Bu | H | - | H | H | $CH_2$ | $CH_2$ | 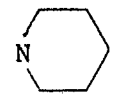 | Oil | cis/trans Ar/N | $^1H$ NMR ($CDCl_3$, 270 MHz); 7.35-7.05 (4H, m), 3.05 and 2.63 (1H, 2xm), 2.55-2.10 (7H, m), 1.98 (2H, m), 1.60 (4H, m), 1.44 (2H, m) and 1.30 and 1.27 (9H, 2xs). |
| 4 | 4-t-Bu | H | $CH_2$ | H | H | $CH_2$ | $CH_2$ | 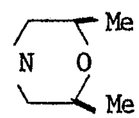 | Oil | cis/trans Ar/N | $^1H$ NMR ($CDCl_3$ 270 MHz); 7.28 (2H, m), 7.08 (2H, m), 3.64 (2H, m), 2.85-2.60 (4H, m), 2.50 (1H, m), 2.20 (2H, m), 2.05 (1H, m), 1.87 (1H, m), 1.58 (1H, m), 1.50 (2H, t, J=10.7 Hz), 1.30 (9H, s) and 1.16 (6H, d, J=6.0 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 5 | 4-i-Pr | H | $CH_2$ | H | H | $CH_2$ | $CH_2$ | 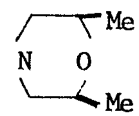 | | | |
| 6a | 4-TMS | H | - | H | H | $CH_2$ | $CH_2$ | 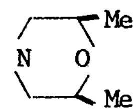 | Oil | cis Ar/N | $^1H$ NMR ($CDCl_3$, 270 MHz); 7.46 (2H, m), 7.24 (2H, m), 3.66 (2H, m), 3.13 (1H, m), 2.72 (2H, d, J=11 Hz), 2.68 (1H, m), 2.49 (2H, m), 1.97 (2H, m), 1.58 (2H, t, J=11 Hz), 1.17 (6H, d, J=6 Hz) and 0.25 (9H, s). |
| 6b | 4-TMS | H | - | H | H | $CH_2$ | $CH_2$ | 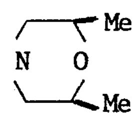 | Oil | cis:trans Ar/N=1:1 | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 7a | 4-*t*-Bu | H | - | H | H | $(CH_2)_2$ | $CH_2$ | 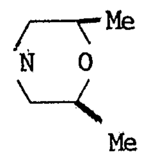 | Oil | *cis* Ar/N | $^1H$ NMR ($CDCl_3$, 400 MHz); 7.34 (2H, m), 7.18 (2H, m), 3.72 (2H, m), 3.03 (1H, m), 2.85 (2H, t, J=11 Hz), 2.64 (1H, m) 2.23 (1H, dt, J=12 and 6 Hz), 2.07 (1H, m), 1.98 (1H, m), 1.78 (2H, m), 1.74 (2H, t, J=11 Hz), 1.60 (1H, m), 1.31 (9H, s), 1.18 (3H, d, J=6 Hz), and 1.16 (3H, d, J=6 Hz). |
| 7b | 4-*t*-Bu | H | - | H | H | $(CH_2)_2$ | $CH_2$ | 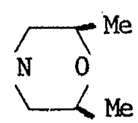 | Oil | *trans* Ar/N | $^1H$ NMR ($CDCl_3$, 400 MHz); 7.32 (2H, m), 7.16 (2H, m), 3.72 (2H, m), 3.20 (1H, m), 2.85 (2H, t, J=11 Hz), 2.74 (1H, m), 2.14 (1H, m), 2.06 (2H, m), 1.88 (1H, m), 1.74 (2H, t, J=11 Hz), 1.64 (2H, m), 1.30 (9H, s), 1.18 (3H, d, J=6 Hz) and 1.16 (3H, d, J= 6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 8a | 4-i-Pr | H | - | H | H | $(CH_2)_2$ | $CH_2$ | 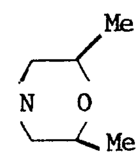 | Oil | cis Ar/N | $^1H$ NMR ($CDCl_3$, 270 MHz); 7.17 (4H, m), 3.71 (2H, m), 3.01 (1H, m), 2.85 (3H, m), 2.61 (1H, m), 2.23 (1H, m), 2.05 (1H, m), 1.97 (1H, m), 1.76 (4H, m), 1.59 (1H, m), 1.24 (6H, d, J=6 Hz), 1.18 (3H, d, J=6 Hz) and 1.16 (3H, d, J=6 Hz). |
| 8b | 4-i-Pr | H | - | H | H | $(CH_2)_2$ | $CH_2$ | 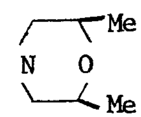 | Oil | trans Ar/N | $^1H$ NMR ($CDCl_3$, 270 MHz); 7.15 (4H, m), 3.70 (2H, m), 3.18 (1H, m), 2.86 (3H, m), 2.73 (1H, m), 2.12 (1H, m), 2.05 (2H, m), 1.90 (1H, m), 1.72 (2H, m), 1.58 (2H, m), 1.24 (6H, d, J=6 Hz), 1.18 (3H, d, J=6 Hz) and 1.15 (3H, d, J=6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 9 | 4-t-Bu | H | - | H | H | $(CH_2)_2$ | $CH_2$ | 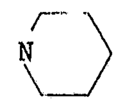 | Oil | cis:trans Ar/N=2:1 | $^1H$ NMR ($CDCl_3$, 270 MHz); 7.30 (2H, m), 7.17 (2H, m), 3.18 and 3.00 (1H, 2xm), 2.74 and 2.64 (1H, 2xm), 2.44 (4H, m), 2.23 (1H, m), 2.04 (2H, m), 1.77 (1H, m), 1.60 (6H, m), 1.44 (2H, m) and 1.30 (9H, s). |
| 10 | 4-t-Bu | H | - | H | H | $(CH_2)_3$ | $CH_2$ | 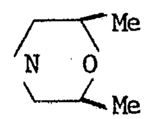 | Oil | trans Ar/N | $^1H$ NMR ($CDCl_3$); 7.33 (2H, m), 7.16 (2H, m), 3.67 (2H, m), 2.98 (1H, m), 2.88 (2H, m), 2.29 (1H, m), 2.08 (1H, m), 1.91 (2H, m), 1.76 (1H, m), 1.35-1.70 (6H, m), 1.29 (9H, s), 1.16 (3H, d, J=7 Hz) and 1.14 (3H, d, J=7 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 11 | 4-t-Bu | H | - | H | H | $(CH_2)_3$ | $CH_2$ | 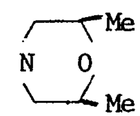 | Oil | cis Ar/N | $^1H$ NMR ($CDCl_3$); 7.31 (2H, m), 7.14 (2H, m), 3.64 (2H, m), 2.78 (2H, m), 2.53 (1H, m), 2.38 (1H, m), 2.08 (1H, m), 1.95 (4H, m), 1.84 (1H, m), 1.30-1.45 (4H, m), 1.28 (9H, s), 1.16 (3H, d, J=7 Hz) and 1.14 (3H, d, J=7 Hz), |
| 12 | 4-i-Pr | H | - | H | H | $(CH_2)_3$ | $CH_2$ | 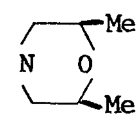 | | | |
| 13 | 3-t-Bu | H | - | H | H | $CH_2$ | $CH_2$ | 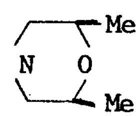 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 14 | 4-t-Bu | H | - | Me | H | $CH_2$ | $CH_2$ | 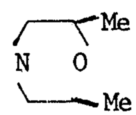 | Oil | cis/trans Ar/N | $^1H$ NMR ($CDCl_3$, 270 MHz); 7.30 (2H, m), 7.30 and 7.10 (2H, 2xm), 3.64 (2H, m), 2.90-1.95 (4H, m), 1.54 and 1.50 (2H, 2xt, J=11 Hz), 1.41 and 1.38 (3H, 2xs), 1.32 and 1.30 (9H, 2xs) and 1.17 (6 H, d, J=6 Hz). |
| 15 | 4-t-Bu | H | - | H | H | $CH_2$ | CHMe | 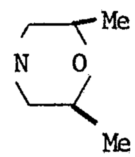 | Oil | | $^1H$ NMR ($CDCl_3$, 270 MHz); 7.35-7.15 (4H, m), 3.72 (2H, m), 2.90-2.20 (6H, m), 1.87 (1H, m), 1.60 (2H, m), 1.31 (9H, s) and 1.20 (9H, m). |
| 16 | 4-t-Bu | H | - | H | H | $CH_2$ | $CH_2$ | 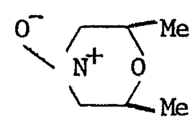 | 107-109 | cis Ar/N | $^1H$ NMR ($CDCl_3$, 400 MHz); 7.34 (4H, m), 4.56 (2H, m), 3.62 (1H, m), 3.12 (1H, m), 3.00 (4H, m), |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 16 (cont/d) | | | | | | | | | | | 2.59 (2H, t, J=11 Hz), 2.44 (2H, m), 1.31 (9H, s) and 1.22 (6H, d, J=6 Hz). |
| 17 | 4-i-Pr | H | - | H | H | $CH_2$ | $CH_2$ | 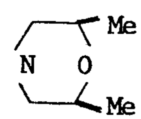 | Oil | cis Ar/N | $^1$H NMR ($CDCl_3$, 270 MHz); 7.17 (4H, m), 3.68 (2H, m), 3.11 (1H, m), 2.87 (1H, sept., J=7 Hz), 2.73 (2H, d, J=11 Hz), 2.64 (1H, m), 2.46 (2H, m), 1.95 (2H, m), 1.58 (2H, t, J=11 Hz), 1.24 (6H, d, J=7 Hz) and 1.18 (6H, d, J=6 Hz). |
| 18 | 4-i-Pr | H | - | H | H | $CH_2$ | $CH_2$ | 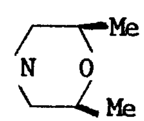 | Oil | cis/trans Ar/N | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 19 | 4-*t*-Bu | H | - | Me | H | $CH_2$ | $CH_2$ | 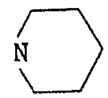 | Oil | *cis* Ar/N | $^1H$ NMR ($CDCl_3$, 400 MHz); 7.25 (2H, m), 7.08 (2H, m), 2.81 (1H, m), 2.30-2.10 (8H, m), 1.55 (4H, m), 1.42 (2H, m), 1.42 (3H, s) and 1.30 (9H, s). |
| 20 | 4-*t*-Bu | H | - | Me | H | $CH_2$ | $CH_2$ | 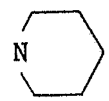 | Oil | *trans* Ar/N | $^1H$ NMR ($CDCl_3$, 400 MHz); 7.36 (2H, m), 7.25 (2H, m), 2.60 (2H, m), 2.47 (1H, m), 2.24 (4H, m), 2.01 (2H, m), 1.57 (4H, m), 1.42 (2H, m), 1.39 (3H, s) and 1.32 (9H, s). |
| 21 | 3-TMS | H | - | H | H | $CH_2$ | $CH_2$ | 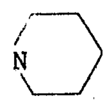 | Oil | *cis*/*trans* Ar/N | $^1H$ NMR ($CDCl_3$, 270 MHz); 7.32 (4H, m), 3.44 and 3.09 (1H, 2xm), 2.89 and 2.63 (1H, 2xm), 2.46 (2H, m), 2.29 (4H, m), 1.98 (2H, m), 1.57 (4H, m), 1.43 (2H, m) and 0.27 and 0.25 (9H, 2xs). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 22 | 3-TMS | H | - | H | H | $CH_2$ | $CH_2$ | 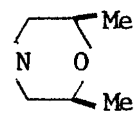 | Oil | cis Ar/N | $^1H$ NMR ($CDCl_3$, 270 MHz); 7.35 (4H, m), 3.67 (2H, m), 3.16 (1H, m), 2.75 (2H, d, J=11 Hz), 2.68 (1H, m), 2.50 (2H, m), 1.98 (2H, m), 1.59 (2H, t, J=11 Hz), 1.17 (6H, d, J=6 Hz) and 0.26 (9H, s). |
| 23 | 3-TMS | H | - | H | H | $CH_2$ | $CH_2$ | 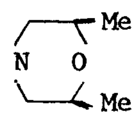 | Oil | cis/trans Ar/N | $^1H$ NMR ($CDCl_3$, 270 MHz); 7.36 (4H, m), 3.67 (2H, m), 3.50 and 3.16 (1H, 2xm), 2.90 and 2.68 (2H, 2xm), 2.78 and 2.75 (2H, 2xd, J=11 Hz), 2.52-1.90 (4H, m), 1.57 (2H, m), 1.17 and 1.14 (6H, 2xd, J=6 Hz) and 0.28 and 0.26 (9H, 2xs). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 24 | 4-Cl | H | - | H | H | $CH_2$ | $CH_2$ | 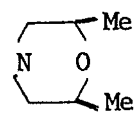 | Oil | cis Ar/N | $^1H$ NMR ($CDCl_3$, 400 MHz), 7.23 (2H, m), 7.14 (2H, m), 3.68 (2H,m), 3.13 (1H, m), 2.72 (2H, d, J=10 Hz), 2.67 (1H, m), 2.49 (2H, m), 1.92 (2H, m), 1.60 (2H, t, J=10 Hz) and 1.19 (6H, d, J=6Hz). |
| 25 | 4-Cl | H | - | H | H | $CH_2$ | $CH_2$ | 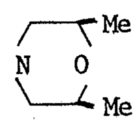 | Oil | cis:trans Ar/N=7:3 | |
| 26 | 3-TMS | H | - | H | H | $CH_2$ | $CH_2$ | 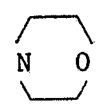 | Oil | cis Ar/N | $^1H$ NMR ($CDCl_3$, 270 MHz); 7.30 (4H, m), 3.73 (4H, m), 3.16 (1H, m), 2.72 (1H, m), 2.50 (2H, m), 2.38 (4H, m), 1.98 (2H, m) and 0.24 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 27 | 3-TMS | H | - | H | H | $CH_2$ | $CH_2$ | 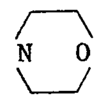 | Oil | cis/trans Ar/N | $^1H$ NMR ($CDCl_3$, 270 MHz); 7.30 (4H, m), 3.73 (4H, m), 3.52 and 3.16 (1H, 2xm), 2.96 and 2.72 (1H, 2xm), 2.55-1.90 (4H, m), 2.38 (4H, m) and 0.26 and 0.24 (9H, 2xs). |
| 28 | 4-t-Bu | H | - | Cl | H | $CH_2$ | $CH_2$ | 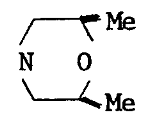 | | | |
| 29 | 4-t-Bu | H | - | Me | H | $CH_2$ | $CH_2$ | 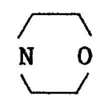 | Oil | cis Ar/N | $^1H$ NMR ($CDCl_3$, 270 MHz); 7.28 (2H, m), 7.06 (2H, m), 3.68 (4H, m), 2.91 (1H, m), 2.70-2.10 (8H, m), 1.45 (3H, s) and 1.29 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 30 | 4-*t*-Bu | H | - | Me | H | $CH_2$ | $CH_2$ | 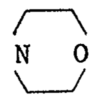 | Oil | *trans* Ar/N | $^1H$ NMR ($CDCl_3$, 270 MHz); 7.34 (2H, m), 7.25 (2H, m), 3.71 (4H, m), 2.60 (3H, m), 2.30 (4H, m), 2.00 (2H, m), 1.39 (3H, m) and 1.32 (9H, s). |
| 31 | 4-*t*-Bu | H | - | H | H | $(CH_2)_2$ | $CH_2$ | 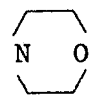 | 38-39 | *cis* Ar/N | $^1H$ NMR ($CDCl_3$, 270 MHz); 7.31 (2H, m), 7.17 (2H, m), 3.75 (4H, m), 3.00 (1H, m), 2.65 (1H, m), 2.50 (4H, m), 2.30-1.50 (6H, m) and 1.31 (9H, s). |
| 32 | 4-*t*-Bu | H | - | H | H | $(CH_2)_2$ | $CH_2$ | 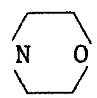 | 44-46 | *trans* Ar/N | $^1H$ NMR ($CDCl_3$, 270 MHz); 7.32 (2H, m), 7.15 (2H, m), 3.75 (4H, m), 3.18 (1H, m), 2.77 (1H, m), 2.50 (4H, m), 2.20-1.50 (6H, m) and 1.31 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 33 | 4-*i*-Pr | H | - | Me | H | $CH_2$ | $CH_2$ | 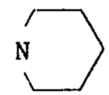 | Oil | *cis/trans* Ar/N | $^1H$ NMR ($CDCl_3$, 270 MHz); 7.08 (4H, m), 2.83 (2H, m), 2.24 (8H, m), 1.50 (6H, m), 1.43 (3H, s) and 1.21 (6H, d, J=6 Hz). |
| 34 | 4-*i*-Pr | H | - | H | H | CHMe | $CH_2$ | 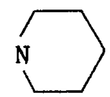 | Oil | Mixture of stereoisomers | $^1H$ NMR ($CDCl_3$, 270 MHz); 7.16 (4H, m), 2.85 (1H, m), 2.60-2.10 (9H, m), 1.70-1.30 (9H, m) and 1.23 (6H, d, J=6 Hz). |
| 35 | 4-*i*-Pr | H | - | H | H | $CH_2$ | $CH_2$ | 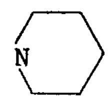 | Oil | *cis* Ar/N | $^1H$ NMR ($CDCl_3$, 270 MHz); 7.16 (4H, m), 3.15 (1H, m), 2.85 (1H, m), 2.60 (1H, m), 2.45 (2H, m), 2.28 (4H, m), 1.94 (2H, m), 1.56 (4H, m), 1.43 (2H, m) and 1.22 (6H, d, J=6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 36 | 4-t-Bu | H | - | H | H | $CH_2$ | $CH_2$ | 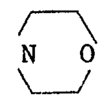 | Oil | cis Ar/N | $^1H$ NMR ($CDCl_3$, 270 MHz); 7.34 (2H, m), 7.20 (2H, m), 3.73 (4H, m), 3.13 (1H, m), 2.72 (1H, m), 2.47 (2H, m), 2.38 (4H, m), 1.87 (2H, m) and 1.31 (9H, s). |
| 37 | 4-TMS | H | - | Me | H | $CH_2$ | $CH_2$ | 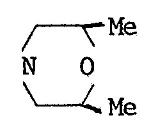 | Oil | cis Ar/N | $^1H$ NMR ($CDCl_3$, 270 MHz); 7.45 (2H, m), 7.13 (2H, m), 3.61 (2H, m), 2.88 (1H, m), 2.64 (2H, d, J=11 Hz), 2.22 (4H, m), 1.56 (2H, d, J=11 Hz), 1.44 (3H, s), 1.15 (6H, d, J=6 Hz) and 0.25 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 38 | 4-TMS | H | - | Me | H | $CH_2$ | $CH_2$ | 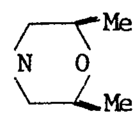 | Oil | trans Ar/N | $^1H$ NMR ($CDCl_3$, 270 MHz); 7.50 (2H, m), 7.34 (2H, m), 3.67 (2H, m), 2.71 (2H, d, J=11 Hz), 2.60 (2H, m), 2.50 (1H, m), 2.01 (2H, m), 1.47 (2H, t, J=11 Hz), 1.41 (3H, s) 1.15 (6H, d, J=6 Hz) and 0.27 (9H, s). |
| 39 | 4-Cl | H | - | H | H | $(CH_2)_2$ | $CH_2$ | 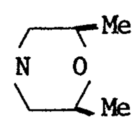 | Oil | cis Ar/N | $^1H$ NMR ($CDCl_3$, 270 MHz); 7.25 (2H, m), 7.18 (2H, m), 3.69 (2H, m), 3.02 (1H, m), 2.85 (2H, m), 2.62 (1H, m), 2.30-1.50 (8H, m), 1.17 (3H, d, J=6Hz) and 1.16 (3H, d, J=6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 40 | 4-Cl | H | - | H | H | $(CH_2)_2$ | $CH_2$ | 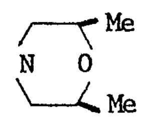 | Oil | trans Ar/N | $^1H$ NMR ($CDCl_3$, 270 MHz); 7.24 (2H, m), 7.14 (2H, m), 3.69 (2H, m), 3.18 (1H, m), 2.83 (2H, m), 2.72 (1H, m), 2.08 (2H, m), 1.90-1.50 (6H, m), 1.18 (3H, d, J=6Hz) and 1.16 (3H, d, J=6Hz). |
| 41 | 4-*t*-Bu | H | - | H | H | $CH_2$ | $CH_2$ | 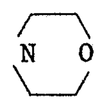 | Oil | cis:trans Ar/N=4:1 | $^1H$ NMR ($CDCl_3$, 270 MHz); 7.34 (2H, m), 7.20 (2H, m), 3.73 (4H, m), 3.48 and 3.13 (1H, 2xm), 2.96 and 2.72 (1H, 2xm), 2.5-2.2 (2H, m), 2.38 (4H, m), 1.97 (2H, m) and 1.31 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 42 | 4-Cl | H | - | Me | H | $CH_2$ | $CH_2$ | 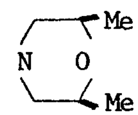 | Oil | cis Ar/N | $^1H$ NMR ($CDCl_3$, 270 MHz); 7.25 (2H, m), 7.06 (2H, m), 3.65 (2H, m), 2.86 (1H, m), 2.70 (2H, d, J=11 Hz), 2.28 (2H, m), 2.15 (2H, m), 1.56 (2H, m), 1.42 (3H, m) and 1.18 (6H, d, J=6 Hz). |
| 43 | 4-TMS | H | - | H | H | $(CH_2)_2$ | $CH_2$ | 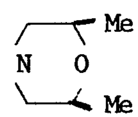 | Oil | cis Ar/N | $^1H$ NMR ($CDCl_3$, 270 MHz); 7.47 (2H, m), 7.25 (2H, m), 3.75 (2H, m), 3.05 (1H, m), 3.87 (2H, t, J=11Hz), 2.66 (1H, m), 2.25 (1H, m), 2.08 (1H, m), 2.00 (1H, m), 1.90-1.60 (5H, m), 1.18 (3H, d, J=6Hz), 1.16 (3H, d, J=6Hz) and 0.25 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 44 | 4-TMS | H | - | H | H | $(CH_2)_2$ | $CH_2$ | 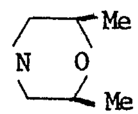 | Oil | trans Ar/N | $^1H$ NMR ($CDCl_3$, 270 MHz); 7.48 (2H, m), 7.22 (2H, m), 3.76 (2H, m), 3.24 (1H, m), 2.88 (2H, d, J=11Hz), 2.78 (1H, m), 2.16 (1H, m), 2.09 (2H, m), 1.90 (1H, m), 1.80-1.60 (4H, m), 1.18 (3H, d, J=6Hz), 1.16 (3H, d, J=6Hz) and 0.25 (9H, s). |
| 45 | 4-i-Bu | H | - | H | H | $CH_2$ | $CH_2$ | 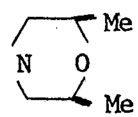 | Oil | cis Ar/N Ar/N | $^1H$ NMR ($CDCl_3$, 270 MHz); 7.15 (2H, m), 7.06 (2H, m), 3.66 (2H, m), 3.11 (1H, m), 3.73 (2H, d, J=11 Hz), 2.66 (1H, m), 2.46 (2H, m), 2.42 (2H, d, J=7 Hz), 1.96 (2H, m), 1.82 (1H, m), 1.57 92H, t, J=11 Hz), 1.17 (6H, d, J=6 Hz) and 0.89 (6H, d, J=7 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 46 | 4-_i_-Pr | H | - | Me | H | $CH_2$ | $CH_2$ | 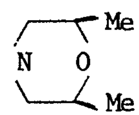 | Oil | _cis_ Ar/N | $^1H$ NMR ($CDCl_3$, 400 MHz); 7.13 (2H, m), 7.06 (2H, m), 3.62 (2H, m), 2.86 (2H, m), 2.70 (2H, d, J=11 Hz), 2.26 (2H, m), 2.18 (2H, m), 1.56 (2H, t, J=11 Hz), 1.44 (3H, s), 1.24 (6H, d, J=7 Hz) and 1.16 (6H, d, J=6 Hz). |
| 47 | 4-_i_-Pr | H | - | Me | H | $CH_2$ | $CH_2$ | 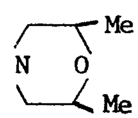 | Oil | _trans_ Ar/N | $^1H$ NMR ($CDCl_3$, 400 MHz); 7.24 (2H, m), 7.19 (2H, m), 3.68 (2H, m), 2.90 (1H, m), 2.82 (2H, d, J=11 Hz), 2.60 (2H, m), 2.50 (1H, m), 2.00 (2H, m), 1.49 (2H, t, J=11 Hz), 1.27 (6H, d, J=6 Hz) and 1.15 (6H, d, J=6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 48 | 4-*t*-Bu | H | – | H | H | $(CH_2)_3$ | – | 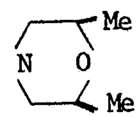 | Oil | *cis* Ar/N | $^1H$ NMR ($CDCl_3$, 400 MHz); 7.27 (2H, m), 7.18 (2H, m), 3.62 (2H, m), 3.00 (2H, m), 2.69 (2H, m), 2.13 (1H, m), 1.98 (1H, m), 1.85-1.55 (6H, m), 1.32 (9H, s), 1.10 (3H, d, J=6 Hz) and 1.04 (3H, d, J=6 Hz). |
| 49 | 4-*i*-Pr | H | – | Me | H | $(CH_2)_2$ | $CH_2$ | 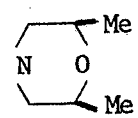 | Oil | *cis* Ar/N | $^1H$ NMR ($CDCl_3$, 400 MHz); 7.22 (2H, m), 7.15 (2H, m), 3.69 (2H, m), 3.86 (4H, m), 2.06 (3H, m), 1.85 (2H, m), 1.74 (3H, m), 1.28 (3H, s), 1.25 (6H, d, J=7 Hz) and 1.18 (6H, d, J=6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 50 | 4-i-Pr | H | - | Me | H | $(CH_2)_2$ | $CH_2$ | 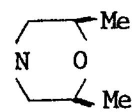 | Oil | trans Ar/N | $^1H$ NMR ($CDCl_3$, 400 MHz), 7.23 (2H, m), 7.17 (2H, m), 3.69 (2H, m), 2.86 (3H, m), 2.55 (1H, m), 2.38 (1H, m), 2.10 (1H, m), 1.85 (2H, m), 1.67 (4H, m), 1.38 (3H, s), 1.25 (6H, d, J=6 Hz), 1.16 (6H, d, J=6 Hz). |
| 51 | 4-t-Bu | H | - | H | H | $(CH_2)_2$ | $CH_2$ | 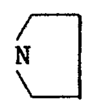 | Oil | cis:trans Ar/N=2:1 | $^1H$ NMR ($CDCl_3$, 270 MHz); 7.23 (2H, m), 7.10 (2H, m), 3.2-2.4 (6H, m), 2.20-1.55 (10H, m) and 1.24 (9H, s). |
| 52 | (2-s-Bu):(4-s-Bu) = 1:1 | H | - | H | H | $(CH_2)_2$ | $(CH_2)_2$ | 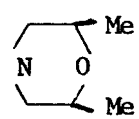 | Oil | trans Ar/N | $^1H$ NMR ($CDCl_3$, 270 MHz); 7.10 (4H, m), 3.70 (2H, m), 2.81 (2H, d, J=11 |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 52 (cont/d) | | | | | | | | | | | Hz), 2.59 (1H, m), 2.46 (1H, m), 2.32 (1H, m), 2.00 (6H, m), 1.70-1.30 (6H, m), 1.22 (3H, d, J=7 Hz), 1.18 (6H, d, J=6 Hz) and 0.82 (3H, t, J=7 Hz). |
| 53 | 4-_t_-Bu | H | - | Me | H | $(CH_2)_2$ | $CH_2$ | 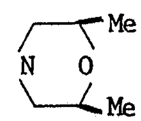 | Oil | _cis_ Ar/N | $^1H$ NMR ($CDCl_3$, 400 MHz); 7.32 (2H, m), 7.24 (2H, m), 3.70 (2H, m), 2.85 (3H, m), 2.05 (4H, m), 1.90-1.55 (4H, m), 1.32 (9H, s), 1.29 (3H, s) and 1.17 (6H, d, J=6 Hz). |
| 54 | 4-_t_-Bu | H | - | Me | H | $(CH_2)_2$ | $CH_2$ | 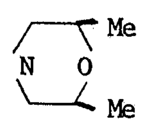 | Oil | _trans_ Ar/N | $^1H$ NMR ($CDCl_3$, 400 MHz); 7.33 (2H, m), 7.23 (2H, m), 3.71 (2H, m), 2.84 (2H, m), 2.56 (1H, m), |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 54 (cont/d) | | | | | | | | | | | 2.36 (1H, m), 2.10 (1H, m), 1.88 (2H, m), 1.65 (4H, m), 1.37 (3H, m), 1.33 (9H, s) and 1.16 (6H, d, J=6 Hz). |
| 55 | 3-_s_-Bu | 5-_s_-Bu | - | H | H | $(CH_2)_2$ | $(CH_2)_2$ | 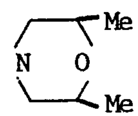 | Oil | | $^1H$ NMR ($CDCl_3$, 270 MHz); 6.85 (3H, m), 3.70 (2H, m), 2.90 (2H, m), 2.70-2.40 ( H, m), 2.25-1.80 (6H, m), 1.75-1.40 (8H, m), 1.23 (6H, d, J=7 Hz), 1.21 (6H, d, J=6 Hz) and 0.83 (6H, t, J=7 Hz). |
| 56 | 4-_t_-Bu | H | - | Cl | H | $(CH_2)_2$ | $CH_2$ | 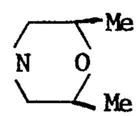 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 57 | 4-s-Bu | H | - | H | H | $(CH_2)_2$ | $CH_2$ | 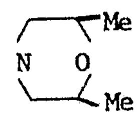 | Oil | cis Ar/N | $^1H$ NMR ($CDCl_3$, 270 MHz); 7.20 (2H, m), 7.10 (2H, m), 3.73 (2H, m), 3.03 (1H, m), 2.86 (2H, m), 2.60 (2H, m), 2.24 (1H, m), 2.04 (2H, m), 1.90-1.50 (7H, m), 1.24 (3H, d, J=7 Hz), 1.18 (3H, d, J=6 Hz), 1.16 (3H, d, J=6 Hz) and 0.82 (3H, t, J=7 Hz). |
| 58 | 4-s-Bu | H | - | H | H | $(CH_2)_2$ | $CH_2$ | 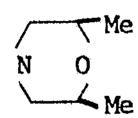 | Oil | trans Ar/N | $^1H$ NMR ($CDCl_3$, 270 MHz); 7.12 (4H, m), 3.72 (2H, m), 3.21 (1H, m), 2.86 (2H, m), 2.77 (1H, m), 2.55 (1H, m), 2.20-1.50 (10H, m), 1.22 (3H, d, J=7 Hz), 1.18 (3H, d, J=6 Hz), 1.16 (3H, d, J=6 Hz) and 0.83 (3H, d, J=7 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 59 | 3-TMS | H | - | H | H | $(CH_2)_2$ | $CH_2$ | 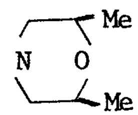 | Oil | cis Ar/N | $^1H$ NMR ($CDCl_3$, 270 MHz); 7.35 (4H, m), 3.74 (2H, m), 3.06 (1H, m), 2.88 (2H, m), 2.64 (1H, m), 2.28 (1H, m), 2.08 (2H, m), 1.90-1.50 (5H, m), 1.19 (3H, d, J=6 Hz), 1.17 (3H, d, J=6 Hz) and 0.26 (9H, s). |
| 60 | 3-TMS | H | - | H | H | $(CH_2)_2$ | $CH_2$ | 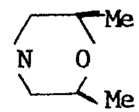 | Oil | cis:trans Ar/N=1:3 | $^1H$ NMR ($CDCl_3$, 270 MHz); 7.30 (4H, m), 3.72 (2H, m), 3.21 and 3.06 (1H, 2xm), 2.95-2.60 (3H, m), 2.35-1.50 (11H, m), 1.19 (3H, d, J=6 Hz), 1.17 (3H, d, J=6 Hz) and 0.27 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 61 | 4-i-Pr | H | - | H | Me | $CH_2$ | $CH_2$ | 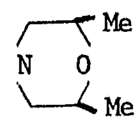 | Oil | cis:trans Ar/N=2:1 | $^1H$ NMR ($CDCl_3$, 270 MHz); 7.17 (4H, m), 3.61 (2H, m), 3.49 and 3.24 (1H, 2xm), 2.95-1.80 (8H, m) and 1.20 (18H, m). |
| 62 | 4-t-Bu | H | - | H | H | $CCl_2$ | $CH_2$ | 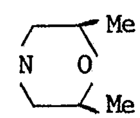 | Oil | | $^1H$ NMR ($CDCl_3$, 270 MHz); 7.27 (2H, m), 7.18 (2H, m), 4.33 (1H, m), 3.96 (1H, m), 3.67 (2H, m), 3.52 (1H, m), 2.97 (1H, m), 2.75 (1H, d, J=11 Hz), 2.56 (1H, d, J=11 Hz), 1.84 (1H, t, J=11 Hz), 1.73 (1H, t, J=11 Hz), 1.33 (9H, s), 1.15 (3H, d, J=6 Hz) and 1.07 (3H, d, J=6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 63 | 4-i-Pr | H | - | H | H | CHMe | $CH_2$ | 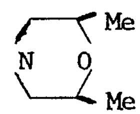 | Oil | Mixture of stereoisomers | $^1H$ NMR ($CDCl_3$, 270 MHz); 7.17 (4H, m), 3.68 (2H, m), 2.90-2.20 (7H, m), 2.00-1.55 (3H, m), 1.24 (6H, d, J=7 Hz) and 1.19 (9H, m). |
| 64 | 4-i-Pr | H | - | H | H | CHMe | $CH_2$ | 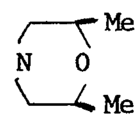 | Oil | trans Ar/N trans Ar/Me | $^1H$ NMR ($CDCl_3$, 400 MHz); 7.17 (4H, m), 3.67 (2H, m), 2.89 (1H, sept., J=7 Hz), 2.78 (1H, d, J=11 Hz), 2.70 (1H, d, J=11Hz), 2.59 (1H, m), 2.40 (1H, m), 2.26 (2H, m), 1.84 (1H, m), 1.70 (1H, t, J=11 Hz), 1.53 (1H, t, J=11 Hz), 1.24 (6H, d, J=7 Hz), 1.21 (3H, d, J = 7 Hz), 1.19 (3H, d, J=6 Hz) and 1.17 (3H, d, J=6 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 65 | 4-i-Pr | H | - | H | H | CHMe | $CH_2$ | 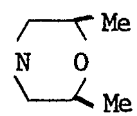 | Oil | cis Ar/N trans Ar/Me | $^1H$ NMR ($CDCl_3$, 270 MHz); 7.19 (4H, m), 3.69 (2H, m), 2.89 (3H, m), 2.67 (2H, d, J=11 Hz), 2.48 (1H, m), 2.21 (1H, m), 1.50 (2H, m), 1.27 (6H, d, J=7 Hz), 1.27 (3H, m), 1.17 (3H, d, J=6 Hz) and 1.15 (3H, d, J=6 Hz). |
| 66 | 4-i-Pr | H | - | H | H | CHMe | $CH_2$ | 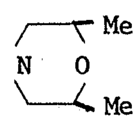 | Oil | cis Ar/N cis Ar/Me | $^1H$ NMR ($CDCl_3$, 270 MHz); 7.18 (2H, m), 7.06 (2H, m), 3.70 (2H, m), 3.35 (1H, m), 2.90 (2H, m), 2.70 (3H, m), 2.23 (2H, m), 1.55 (2H, m), 1.26 (6H, d, J=7 Hz), 1.17 (3H, d, J=6 Hz), 1.15 (3H, d, J=6 Hz) and 0.74 (3H, d, J=7 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 67 | 4-_i_-Pr | H | - | H | H | CHMe | CHMe | 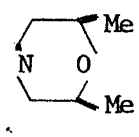 | Oil | Mixture of stereoisomers | $^1H$ NMR ($CDCl_3$, 270 MHz); 7.10 (4H, m), 3.68 (2H, m), 2.95-2.20 (7H, m), 1.60 (2H, m) and 1.20 and 0.66 (18H, 2xm). |
| 68 | 4-_s_-Bu | H | - | H | H | $CH_2$ | $CH_2$ | 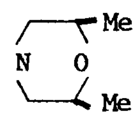 | Oil | _cis_ Ar/N | $^1H$ NMR ($CDCl_3$, 270 MHz); 7.16 (2H, m), 7.09 (2H, m), 3.67 (2H, m), 3.11 (1H, m), 2.73 (2H, d, J=11 Hz), 2.66 (1H, m), 2.57 (1H, m), 2.46 (2H, m), 1.96 (2H, m), 1.60 (4H, m), 1.22 (3H, d, J=6 Hz), 1.17 (6H, d, J=6 Hz) and 0.81 (3H, t, J=7 Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 69 | 4-s-Bu | H | - | H | H | $CH_2$ | $CH_2$ | 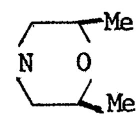 | Oil | cis:trans Ar/N=1:4 | $^1H$ NMR ($CDCl_3$, 270 MHz); 7.17 (4H, m), 3.69 (2H, m), 3.47 and 3.11 (1H, 2xm), 3.00-2.20 (8H, m), 1.55 (4H, m), 1.20 (9H, m) and 0.83 (3H, t, J=7 Hz). |
| 70 | 4-Cl | H | $CH_2$ | H | H | $CH_2$ | $CH_2$ | 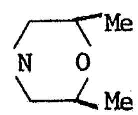 | Oil | cis/trans Ar/N | $^1H$ NMR ($CDCl_3$, 270 MHz); 7.22 (2H, m), 7.04 (2H, m), 3.66 (2H, m), 2.80-2.10 (10H, m), 1.55 (2H, m) and 1.17 (6H, d, J=6 Hz). |
| 71 | 4-Cl | H | $CH_2$ | H | H | $(CH_2)_2$ | $CH_2$ | 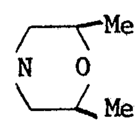 | Oil | | $^1H$ NMR ($CDCl_3$, 270 MHz); 7.25 (2H, m), 7.09 (2H, m), 3.73 (2H, m), 2.84 (2H, m), 2.70-2.40 (3H, m). 2.20-1.40 (9H, m), 1.15 (6H, d, J=6Hz). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 72 | 4-t-Bu | H | - | H | H | $CH_2CHMe$ | $CH_2$ | 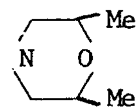 | Oil | 1RS, 3RS, 5SR | $^1H$ NMR ($CDCl_3$, 270 MHz); 7.33 (2H, m), 7.18 (2H, m), 3.73 (2H, m), 3.10 (1H, m), 2.82 (2H, m), 2.48 (2H, m), 2.33 (1H, m), 2.13 (1H, m), 1.90-1.60 (4H, m), 1.31 (9H, s), 1.15 (6H, m) and 1.02 (3H, d, J=7Hz). |
| 73 | 4-t-Bu | H | - | H | H | $CH_2CHMe$ | $CH_2$ | 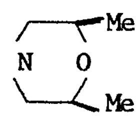 | Oil | 1RS, 3SR, 5RS:1RS, 3RS, 5RS = 3:2 | $^1H$ NMR ($CDCl_3$, 270 MHz); 7.34 (2H, m), 7.15 (2H, m), 3.74 (2H, m), 3.20-1.60 (11H, m), 1.31 (9H, s) and 1.20 (9H, m). |
| 74 | 4-t-Bu | H | - | H | H | $CH_2$ | $CH_2$ | 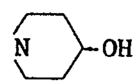 | 99°C | cis:trans Ar/N=4:1 | $^1H$ NMR ($CDCl_3$, 270 MHz); 7.42 (2H, m), 7.37 (2H, m), 4.24 (1H, m), 3.60-2.10 ( H, m), 1.91 (2H, d, J=11Hz) and 1.29 (9H, s). |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 75 | 4-t-Bu | H | - | H | H | $CMe_2$ | $CH_2$ | 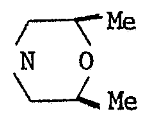 | | | |
| 76 | 4-t-Bu | H | - | H | H | $(CH_2)_2$ | $CH_2$ | 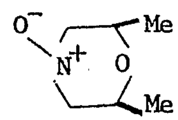 | Gum | cis/trans Ar/N | $^1H$ NMR ($CDCl_3$, 270 MHz); 7.32 (2H, m), 7.15 (2H, m), 4.52 (2H, m), 3.40 (2H, m), 3.40-1.60 (12H, m), 1.32 (9H, m) and 1.20 (6H, m). |
| 77 | 4-i-Pr | H | - | H | H | $CH_2$ | $CH_2$ | 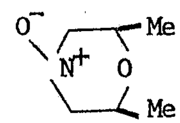 | Gum | cis/trans Ar/N | $^1H$ NMR ($CDCl_3$, 270 MHz); 7. ( H, m), 4.53 (2H, m), 3.72 (2H, m), 3.40-1.60 (13H, m), 1.20 (12H, m). |
| 78 | 4-t-Bu | H | - | H | H | $(CH_2)_2$ | CHMe | 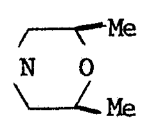 | | | |

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 79 | 4-t-Bu | H | - | H | H | $CHMeCH_2$ | $CH_2$ | 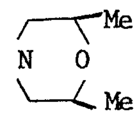 | | Me at 4 position on cyclopentane | |
| 80 | 4-i-Pr | H | - | H | H | $(CH_2)_2$ | CHMe | 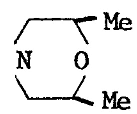 | | | |
| 81 | 4-t-Bu | H | - | H | H | $(CH_2)_2$ | CHOH | 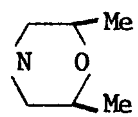 | | | |
| 82 | 4-Me | 3-Me | - | H | H | $(CH_2)_2$ | $CH_2$ | 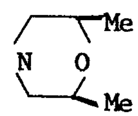 | | | |
| 83 | 4-t-Bu | H | - | H | H | $(CH_2)_2$ | $(CH_2)_2$ | 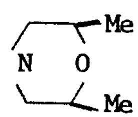 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 84 | 4-t-Bu | H | - | H | H | $(CH_2)_2$ | CHCl | 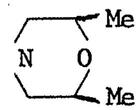 | | | |
| 85 | 4-t-Bu | H | - | H | H | $CH_2CHCl$ | $CH_2$ | 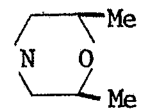 | | | |
| 86 | 4-t-Bu | H | - | H | H | $CH_2CHOH$ | $CH_2$ | 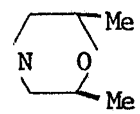 | | | |
| 87 | 4-t-Bu | H | - | H | H | CHOH | $CH_2$ | 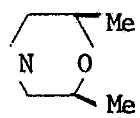 | | | |
| 88 | 4-t-Bu | H | - | H | H | $CH_2$ | $CH_2$ | 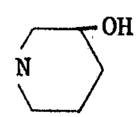 | | | |

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 89 | 4-t-Bu | H | - | H | H | $CH_2$ | $CH_2$ | 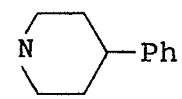 | | | |
| 90 | 4-t-Bu | H | - | H | H | $CH_2$ | $CH_2$ | 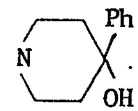 | | | |
| 91 | 4-t-Bu | H | - | H | H | $CH_2$ | $CH_2$ | 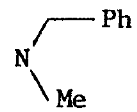 | | | |
| 92 | 4-t-Bu | H | - | H | H | $(CH_2)_2$ | $CH_2$ | 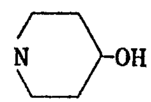 | | | |
| 93 | 4-t-Bu | H | - | H | H | $(CH_2)_2$ | $CH_2$ | 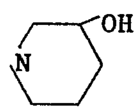 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 94 | 4-t-Bu | H | - | H | H | $(CH_2)_2$ | $CH_2$ | 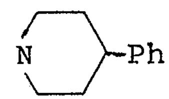 | | | |
| 95 | 4-t-Bu | H | - | H | H | $(CH_2)_2$ | $CH_2$ | 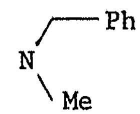 | | | |
| 96 | 4-i-Pr | Me | - | H | H | $CH_2$ | $CH_2$ | 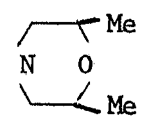 | | | |
| 97 | 4-i-Pr | Me | - | H | H | $(CH_2)_2$ | $CH_2$ | 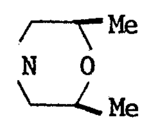 | | | |
| 98 | $-CH_2-CH_2-CH_2-$ at 3 and 4 positions | | - | H | H | $CH_2$ | $CH_2$ | 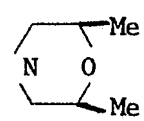 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 99 | $-CH_2CH_2CH_2-$ at 3 and 4 positions | | – | H | H | $(CH_2)_2$ | $CH_2$ | 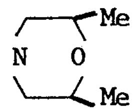 | | | |
| 100 | 4-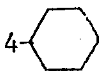 | H | – | H | H | $CH_2$ | $CH_2$ | 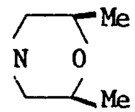 | | | |
| 101 | 4-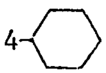 | H | – | H | H | $(CH_2)_2$ | $CH_2$ | 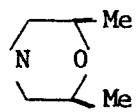 | | | |
| 102 | 4-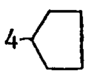 | H | – | H | H | $CH_2$ | $CH_2$ | 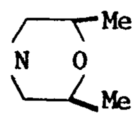 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 103 | 4-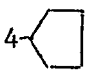 | H | – | H | H | $(CH_2)_2$ | $CH_2$ | 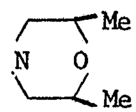 | | | |
| 104 | 4-t-Bu | H | – | H | H | $CH_2$ | CHEt | 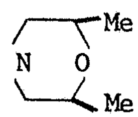 | | | |
| 105 | 4-i-Pr | H | – | H | H | $CH_2$ | CHEt | 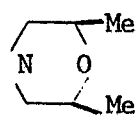 | | | |
| 106 | 4-t-Bu | H | – | H | H | $(CH_2)_2$ | CHEt | 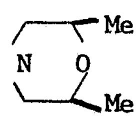 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 107 | 4-i-Pr | H | – | H | H | $(CH_2)_2$ | CHEt | 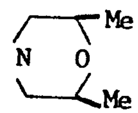 | | | |
| 108 | 4-i-Pr | H | – | H | H | $(CH_2)_2$ | $(CH_2)_2$ | 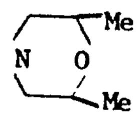 | | | |
| 109 | 4-O-t-Bu | H | – | H | H | $CH_2$ | $CH_2$ | 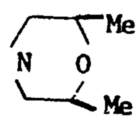 | | | |
| 110 | 4-O-t-Bu | H | – | H | H | $(CH_2)_2$ | $CH_2$ | 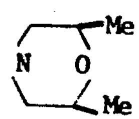 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 111 | 4-OMe | H | - | H | H | $CH_2$ | $CH_2$ | 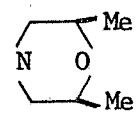 | | | |
| 112 | 4-OMe | H | - | H | H | $(CH_2)_2$ | $CH_2$ | 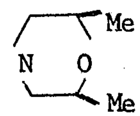 | | | |
| 113 | 4-Cl | H | - | H | H | $(CH_2)_2$ | $(CH_2)_2$ | 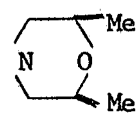 | | | |
| 114 | 4-TMS | H | - | H | H | $(CH_2)_2$ | $(CH_2)_2$ | 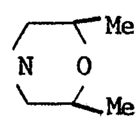 | | | |

TABLE I (CONT/D)

| COMPOUND NO. | X | Y | $CR^1R^2$ | $R^3$ | $R^4$ | Q | Z | $NR^5R^6$ | M.P. | COMMENTS | NMR DATA |
|---|---|---|---|---|---|---|---|---|---|---|---|
| 115 | 3-TMS | H | - | H | H | $(CH_2)_2$ | $(CH_2)_2$ | 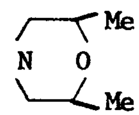 | | | |

Compounds of general formula (I):

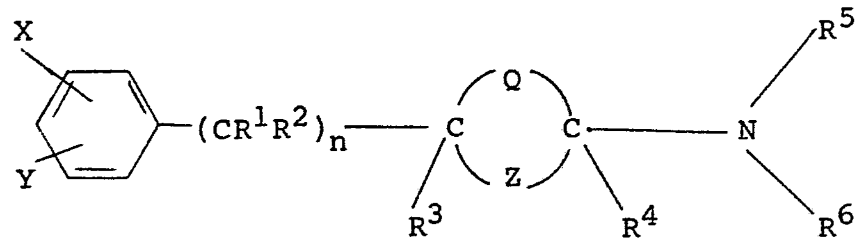

(I)

wherein $R^1$ to $R^3$, $R^5$, $R^6$, Q, X, Y, Z and n are as defined above and $R^4$ is hydrogen can be prepared by reductive amination of compounds of general formula (II):

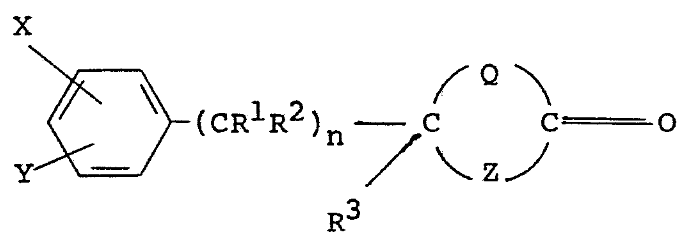

(II)

wherein $R^1$ to $R^3$, Q, X, Y, Z and n are as defined above with an amine of general formula (III):

$HNR^5R^6$

(III)

wherein $R^5$ and $R^6$ are as defined above with a reducing agent, for instance formic acid, sodium cyanoborohydride or tetrabutylammonium cyanoborohydride.

Compounds of general formula (II) wherein $Q=Q_1$ ($R^7=R^8=$hydrogen) and $Z=Z_1$ may be prepared by treatment of compounds of general formula (IV):

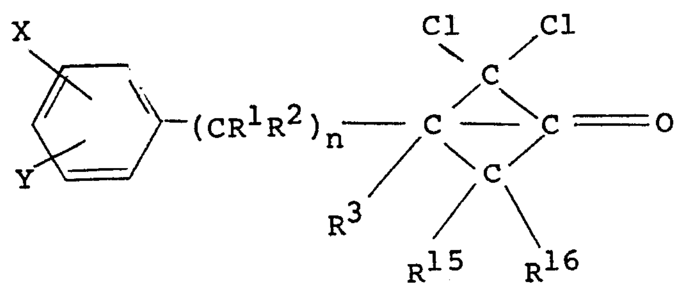

(IV)

wherein $R^1$ to $R^3$, $R^{15}$, $R^{16}$, X, Y and n are as defined above with a dechlorinating agent such as tributyltin hydride or zinc in acetic acid.

Compounds of general formula (IV) may be prepared by treatment of a compound of general formula (V):

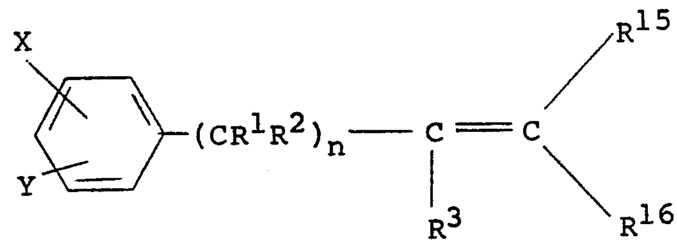

(V)

wherein $R^1$ to $R^3$, $R^{15}$, $R^{16}$, X, Y and n are as defined above with dichloroketene generated, for instance, by treatment of trichloroacetyl chloride with zinc or dichloroacetyl chloride with a tertiary amine such as triethylamine.

As an alternative compounds of general formula (II) wherein $Q=Q_1$ ($R^7$=$R^8$=hydrogen) and $Z=Z_1$ may be prepared by treatment of compounds of general formula (V) with ketene generated, for instance, by thermal treatment of acetone.

Compounds of general formula (V) may be prepared by methods set out in the literature. For instance, in cases where n = 0 compounds of general formula (V) may be prepared by treatment of compounds of general formula (VI):

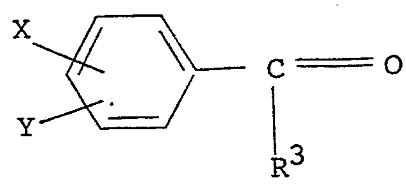

(VI)

wherein $R^3$, X and Y are as defined above with a phosphorane of general formula (VII):

$$Ph_3P=CR^{15}R^{16}$$

(VII)

wherein $R^{15}$ and $R^{16}$ are as defined above under the conditions of the Wittig reaction, or a variation thereof.

Compounds of general formula (VI) may be prepared by methods set out in the literature.

Compounds of general formula (V) wherein n = 1 and $R^1 = R^2$ = hydrogen may be prepared, for instance, by treatment of a Grignard reagent of general (VIII):

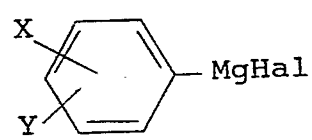

(VIII)

wherein X and Y are as defined above and Hal is chlorine, bromine or iodine with an allyl bromide of general formula (IX):

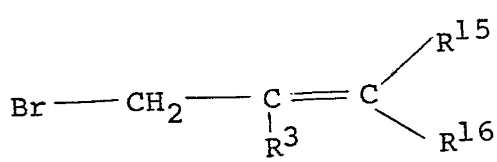

(IX)

wherein $R^3$, $R^{15}$ and $R^{16}$ are as defined above.

Compounds of general formula (VIII) and (IX) may be prepared by methods set out in the literature.

As an alternative compounds of general formula (I) wherein $Q = Q_1$ and $Z = Z_1$ and $R^4$ = hydrogen may be prepared by treatment of a compound of general formula (V) wherein $R^1$ to $R^3$, $R^{15}$, $R^{16}$, X, Y and n are as defined above with amide of general formula (X):

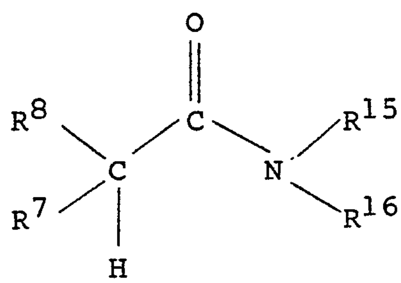

(X)

in the presence of trifluoromethanesulphonic anhydride and an amine base such as lutidine or collidine followed by in situ treatment with a reducing agent such as tetrabutylammonium cyanoborohydride.

Compounds of general formula (X) may be prepared by methods set out in the literature.

As a further alternative compounds of general formula (I) wherein $Q = Q_1$ and $Z = Z_1$ and $R^4$ = alkyl may be prepared by treatment of a compound of general formula (V) with a compound of general formula (X) as described above followed by in situ treatment with a Grignard reagent of general formula (XI):

$$R^4\ Mg\ Hal$$

(XI)

wherein $R^4$ and Hal are as defined above.

Compounds of general formula (II) wherein $Z = Z_0$ and $R^3$ = hydrogen may be prepared by treatment of a compound of general formula (XII):

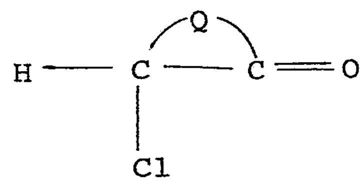

(XII)

wherein Q is as defined above with a Grignard reagent of general formula (XIII):

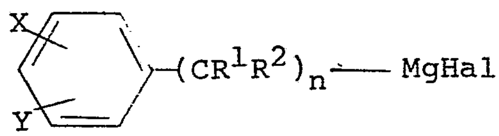

(XIII)

wherein $R^1$, $R^2$, X, Y, n and Hal are as defined above.

Compounds of general formula (XII) may be prepared by treatment of compounds of general formula (XIV):

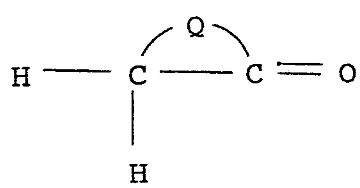

(XIV)

wherein Q is as defined above with chlorine as described in the literature.

Compounds of general formula (XIV) may be prepared by methods set out in the literature.

As an alternative compounds of general formula (II) in which Z = $Z_0$ may be prepared by oxidation of compounds of general formula (XV):

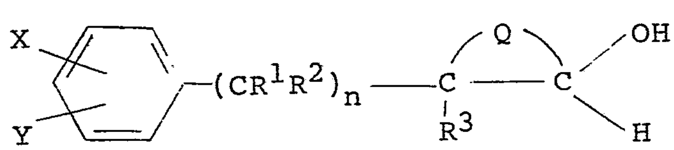

(XV)

wherein $R^1$ to $R^3$, Q, X, Y and n are as defined above.

Compounds of general formula (XV) wherein $R^3$ = hydrogen may be prepared by treatment of an epoxide of general formula (XVI):

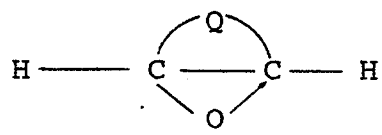

(XVI)

wherein Q is as defined above with a Grignard reagent of general formula (XIII) in the presence of a copper (I) catalyst.

Compounds of general formula (XVI) can be prepared by treatment of compounds of general formula (XVII):

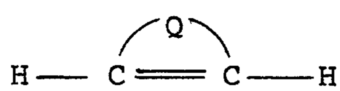

(XVII)

wherein Q is as defined above with a peroxyacid such as peroxyacetic acid or m-chloro-peroxybenzoic acid.

Compounds of general formula (XVII) may be prepared by methods set out in the literature.

Compounds of general formula (II) wherein Z = $Z_1$ may be prepared by treatment of enones of general formula (XVIII):

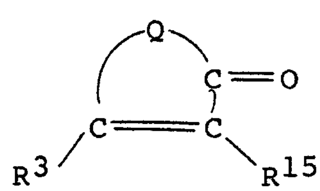

(XVIII)

wherein $R^3$, $R^{15}$ and Q are as defined above with an organocuprate reagent of general formula (XIX):

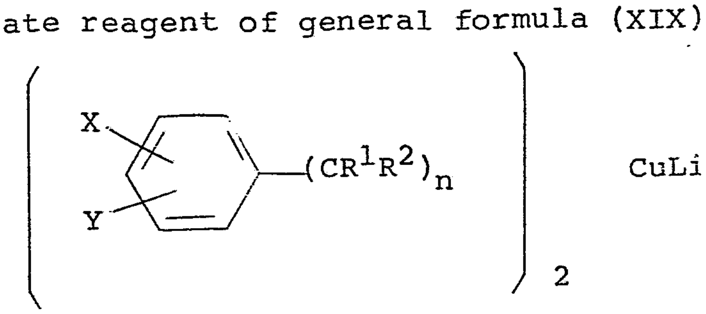

(XIX)

wherein $R^1$, $R^2$, X, Y and n are as defined above or an organozinc reagent of general formula (XX):

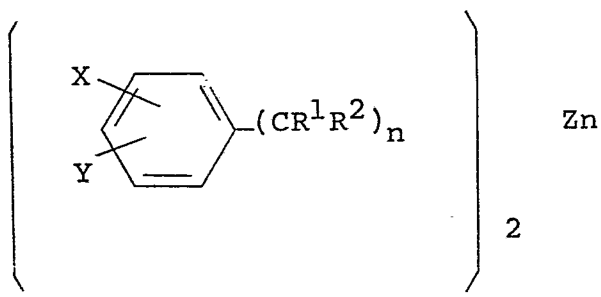

(XX)

wherein $R^1$, $R^2$, X, Y and n are as defined above, optionally in the presence of a catalytic amount of nickel acetylacetonate.

Compounds of general formula (XVIII) can be prepared as set out in the literature.

As an alternative compounds of general formula (II) wherein $Z = Z_1$ may be prepared by treatment of a compound of general formula (XXI):

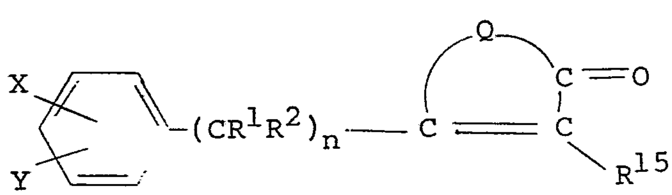

(XXI)

wherein $R^1$, $R^2$, $R^{15}$, Q, X, Y and n are as defined above with an organocuprate reagent of general formula (XXII):

$$(R^3)_2\ CuLi$$

(XXII)

wherein $R^3$ is as defined above. For cases where $R^3$ = $R^{16}$ = hydrogen compounds of general formula (II) can be prepared by hydrogenation of compounds of general formula (XXI) over the usual hydrogenation catalysts such as palladium on charcoal.

Compounds of general formula (XXI) can be prepared by treating compounds of general formula (XXIII):

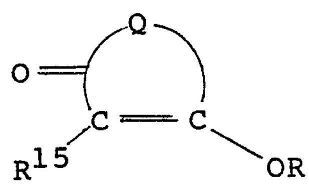

(XXIII)

wherein $R^{15}$ and Q are as defined above and R = alkyl with an organolithium reagent of general formula (XXIV):

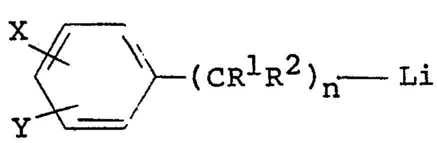

(XXIV)

wherein $R^1$, $R^2$, X, Y and n are as defined above or a Grignard reagent of general formula (XIII) followed by acid catalysed rearrangement.

Compounds of general formula (XXIII) can be prepared by methods set out in the literture.

 

Compounds of general formula (I) wherein n = 1 can be prepared from compounds of general formula (XXV):

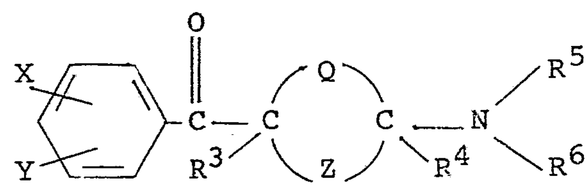

(XXV)

wherein $R^3$ to $R^6$, Q, X, Y and Z are as defined above by a variety of methods. For instance, for cases where $R^1 = R^2$ = hydrogen compounds of general formula (I) wherein n = 1 can be prepared by treatment of compounds of general formula (XXV) with a reducing agent, such as triethylsilane in trifluoroacetic acid. For cases where $R^1$ = hydrogen and $R^2$ = alkyl compounds of general formula (I) wherein n = 1 can be prepared by treatment of compounds of formula (XXV) with a Grignard reagent of a general formula (XXVI):

$R^2$ Mg Hal

(XXVI)

wherein $R^2$ and Hal are as defined above followed by treatment with a reducing agent. For cases where $R^2$ = hydrogen or alkyl and $R^1$ = fluorine, chlorine or bromine compounds of general formula (I) wherein n = 1 can be prepared by treatment of compounds of general formula (XXV) with either sodium borohydride (for $R^2$ = hydrogen) or a Grignard reagent of general formula (XXVI) (for $R^2$ = alkyl) followed by a halogenating agent. For example, thionyl chloride ($R^1$ = chlorine), phosphorus tribromide ($R^1$ = bromine) or diethylamino sulphurtrifluoride ($R^1$ = fluorine).

Compounds of general formula (XXV) wherein $R^4$ = hydrogen can be prepared by treatment of compounds of general formula (XXVII):

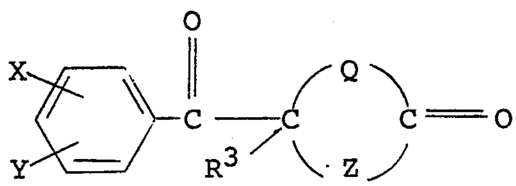

(XXVII)

wherein $R^3$, Q, X, Y and Z are as defined above by an amine of general formula (III) under reductive conditions as described above.

Compounds of general formula (XXVII) wherein Z = $Z_1$ and $R^{16}$ = hydrogen can be prepared by treatment of compounds of general formula (XVIII) with a compound of general formula (XXVIII):

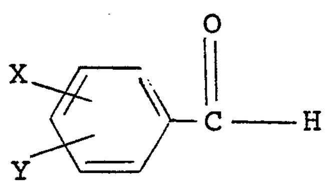

(XXVIII)

wherein X and Y are as defined above in the presence of a cyanide catalyst.

Compounds of general formula (XXVIII) can be prepared by methods set out in the literature.

As a further alternative compounds of general formula (I) wherein $R^3$ = hydrogen can be prepared by treating compounds of general formula (I) wherein $R^3$ = chlorine or bromine with tributyltin hydride.

Compounds of general formula (I) wherein $R^3$ = chlorine or bromine can be prepared by treatment of compounds of general formula (XXIX):

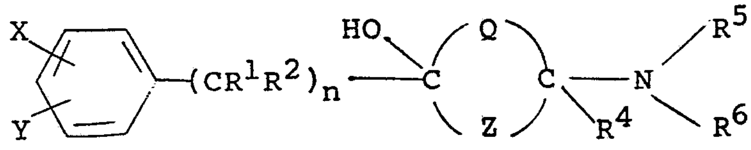

(XXIX)

wherein $R^1$, $R^2$, $R^4$ to $R^6$, Q, X, Y, Z and n are as defined above with the usual halogenating agents. For example, thionyl chloride or phosphorus trichloride ($R^3$ = chlorine) or phosphorus tribromide ($R^3$ = bromine).

As an alternative compounds of general formula (I) wherein n = 0 and $R^3$ = hydrogen can be prepared by treatment of compounds of general formula (XXIX) with a reducing agent such as triethylsilane in trifluoroacetic acid, or by hydrogenation.

Compounds of general formula (XXIX) can be prepared by treating compounds of general formula (XXX):

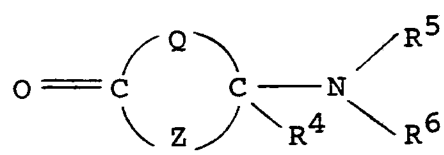

(XXX)

wherein $R^4$ to $R^6$, Q and Z are as defined above with a Grignard reagent of general formula (XIII) or an organolithium reagent of general formula (XXIV).

Compounds of general formula (XXX) wherein Z = $Z_1$ and $R^{16}$ = hydrogen can be prepared by treatment of compounds of general formula (XXXI):

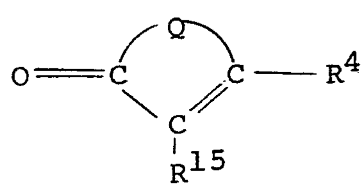

(XXXI)

wherein $R^4$, $R^{15}$ and Q are as defined above with an amine of general formula (III).

Compounds of general formula (XXXI) can be prepared by methods set out in the literature.

The compounds, their salts and N-oxides are active fungicides, particularly against the diseases *Puccinia recondita*, *Puccinia striiformis* and other rusts on wheat, *Puccinia hordei*, *Puccinia striiformis* and other rusts on barley, and rusts on other hosts e.g. coffee, apples, vegetables and ornamental plants

*Erysiphe graminis* (powdery mildew) on barley and wheat and other powdery mildews on various hosts such as *Sphaerotheca fuliginea* on cucurbits (e.g. cucumber), *Podosphaera leucotricha* on apples and *Uncinula necator* on vines

*Helminthosporium* spp., e.g. *Pyrenophora teres*

*Rhynchosporium* spp., *Septoria* spp. and *Rhizoctonia* spp. e.g. *R. cerealis*

*Cercospora arachidicola* on peanuts and other *Cercospora* species on for example sugar beet, bananas and soya beans

*Venturia inaequalis* (scab) on apples.

Some of the compounds have also shown a broad range of activities against fungi *in vitro*. They have activity against various post-harvest diseases on fruit (e.g. *Penicillium diagatum* and *italicum* on oranges *Gloeosporium musarum* on bananas and *Botrytis cinerea* on grape). Further some of the compounds are active as seed dressings against :

*Fusarium* spp., *Septoria* spp., *Tilletia* spp. (i.e. bunt, a seed borne disease of wheat), *Ustilago* spp. and *Helminthosporium* spp. on cereals.

The compounds can move acropetally in the plant tissue. Moreover, the compounds can be volatile enough to be active in the vapour phases against fungi on the plant.

They may also be useful as industrial (as opposed to agricultural) fungicides, e.g. in the prevention of fungal attack on wood, hides, leather and especially paint films.

The compounds may be used as such for fungicidal purposes but are more conveniently formulated into compositions for such usage. The invention thus provides a fungicidal composition comprising a compound of general formula (I) as hereinbefore defined, or a salt, thereof; and, optionally, a carrier or diluent.

The invention also provides a method of combating a fungi, which comprises applying to a plant, to seed of a plant, or to the locus of the plant or seed, a compound, or salt, thereof, as hereinbefore defined.

The compounds and their salts can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be performed granules suitable for application to the soil without further treatment. These granules can be made either by impregnating pellets of filler with the active ingredient or be pelleting a mixture of the active ingredient and powder filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or

emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in microencapsulated form. They may also be formulated in biodegradable polymeric formulations to obtain a slow, controlled release of the active substance.

By including suitable additives, for example additives for improving the distribution, adhesive powder and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt thereof.

The compositions may also be in the form of liquid preparations for use as dips or sprays which are generally aqueous dispersions or emulsions containing the active ingredient in the presence of one or more surfactants e.g.

wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonate aromatic compounds (for example sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydride, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The

concentrates may conveniently contain up to 95%, suitably 10-85%, for example 25-60%, by weight of the active ingredient(s). These concentrates suitably contain organic acids (e.g. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal activity or compounds having plant growth regulating, herbicidal or insecticidal activity.

The other fungicidal compound can be, for example, one which is capable of combating ear diseases of cereals (e.g. wheat such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are carbendazim, benomyl, thiophanatemethyl, thiabendazole, fuberidazole, etridazole, dichlorofluanid, cymoxanil, oxadixyl, ofurace, metalaxyl, furalaxyl, benalaxyl, fosetyl-aluminium, fenarimol, iprodione, procymidione, vinclozolin,

penconazole, myclobútanil, RO151297, pyrazophos, ethirimol, ditalimofos, tridemorph, triforine, nuarimol, triazbutyl, guazatine, propiconazole, prochloraz, flutriafol, hexaconazole i.e. the chemical 1-(1,2,4-triazol-1-yl)-2-(2,4-dichlorophenyl)hexan-2-ol, DPX H6573 (1-((bis-4-fluorophenyl)methylsilyl)methyl)-1H-1,2,4-triazole, triadimefon, triadimenol, diclobutrazol, fenpropimorph, fenpropidin, chlorozolinate, diniconazole, imazilil, fenfuram, carboxin, oxycarboxin, methfuroxam, dodemorph, BAS 454, blasticidin S, kasugamycin, edifenphos, kitazin P, cycloheximide, phthalide, probenazole, isoprothiolane, tricyclazole, pyroquilan, chlorbenzthiazone, neoasozin, polyoxin D, validamycin A, repronil, fluotolanil, pencycuron, diclomezine, phenazin oxide, nickel dimethyldithiocarbamate, techlorthalem, bitertanol, bupirimate, etaconazole, strptomycin, cypofuram, biloxazol, quinomethionate, dimethirimol, 1-(2-cyano-2-methoxyimino-acetyl)-3-ethyl urea, fenapanil, tolclofosmethyl, pyroxyfur, polyram, maneb, mancozeb, captafol, chlorothalonil, anilazine, thiram, captan, folpet, zineb, propineb, sulphur, dinocap, binapactryl, nitrothalisopropyl, dodine, dithianon, fentin hydroxide, fentin acetate, tecnazene, quintozene, dichloran, copper containing compounds such as copper oxychloride, copper sulphate and Bordeaux mixture, and organomercury compounds.

The compounds of general formula (I) can be mixed with soil, peat or other rooting media for the protection of plants against seed-borne, soil-borne or foliar fungal diseases.

Suitable insecticides which may be incorporated in the composition of the invention include pirimicarb, dimethoate, demeton-s-methyl, formothion, carbaryl, isoprocarb, XMC, BPMC, carbofuran, carbosulfan, diazinon, fenthion, fenitrothion, phenothoate, chlorpyrifos,

isoxathion, propaphos, monocrotophos, buprofezin, ethroproxyfen and cycloprothrin.

Plant growth regulating compounds for use in the invention compositions are compounds which control weeds or seedhead formation, or selectively control the growth of less desirable plants (e.g. grasses).

Examples of suitable plant growth regulating compounds for use with the invention compositions are the gibberellins (e.g. $GA_3$, $GA_4$ or $GA_7$), the auxins (e.g. indoleacetic acid, indolebutyric acid, naphthoxyacetic acid or naphthylacetic acid), the cytokinins (e.g. kinetin, diphenylurea, benzimidazole, benzyladenine or benzylaminopurine), phenoxyacetic acids (e.g. 2,4-D or MCPA), substituted benzoic acids (e.g. triiodobenzoic acid), morphactins (e.g. chlorfluoroecol), maleic hydrazide, glyphosate, glphosine, long chain fatty alcohols and acids, dikegulac, paclobutrazol, flurprimidol, fluoridamid, mefluidide, substituted quaternary ammonium and phosphonium compounds (e.g. chloromequat chlorphonium or mepiquatchloride), ethephon, carbetamide, methyl-3,6-dichloroanisate, daminozide, asulam, abscisic acid, isopyrimil, 1-(4-chlorophenyl)-4,6-dimethyl-2-oxo-1,2-dihydropyridine-3-carboxylic acid, hydroxybenzonitriles (e.g. bromoxynil), difenzoquat, benzoylpropethyl 3,6-dichloropicolinic acid, fenpentezol, inabenfide, triapenthenol and tecnazene.

The following Examples illustrate the invention; the temperatures are given in degrees Centigrade (°C).

## EXAMPLE 1

This Example illustrates the preparation of Z and E 3-(4-t-butylphenyl)-1-[4-(cis 2,6-dimethylmorpholino)]-cyclobutane (Compounds Nos. 1 and 2 of Table I).

Dimethyl sulphoxide (90ml) was added to hexane - washed sodium hydride (6.0g of a 50% dispersion in oil; 124mmol) under argon and the stirred suspension heated at 70°C for 2 hours. The mixture was then cooled to 0°C and tetrahydrofuran (100ml) added, followed by methyltriphenylphosphonium bromide (46.2g, 130mmol) in small portions (5g). After stirring for 1 hour 4-t-butylbenzaldehyde (20g, 124mmol) was slowly added. After stirring for 24 hours, water was added and the mixture extracted with hexane (x2). The extracts were repeatedly washed with water, dried ($Na_2SO_4$) and evaporated in vacuo. Chromatography ($SiO_2$, hexane) gave (4-t-butylphenyl)ethene (14.1g, 71%).

Trichloroacetyl chloride (12.5ml, 112 mmol) in dry ether (50ml) was added to (4-t-butylphenyl)ethene (12.0g, 75 mmol) and zinc powder (9.8g, 150mmol) in dry ether (300ml) under argon in a sonic bath over 30 minutes. After 1 hour zinc powder (2.0g, 30mmol) was added. After a further 1 hour water was cautiously added, and the mixture filtered through celite. The filtrate was washed with water, saturated sodium bicarbonate solution and water, dried ($Na_2SO_4$) and evaporated in vacuo. Filtration through silica gel eluting with ether-hexane (10:90) gave 3-(4-t-butylphenyl)-2,2-dichlorocyclobutanone (17.0g, 84%).

Azobisisobutyronitrile (50mg) and 3-(4-t-butylphenyl)-2,2-dichloro-cyclobutanone (5.36g, 19.8mmol) in cyclohexane (12ml) were slowly added to a refluxing solution of tri-n-butyltin hydride (11.7ml 43.7mmol) in cyclohexane (20ml) under argon. After 2 hours the mixture was cooled evaporated in vacuo and chromatographed [$SiO_2$, hexane-methyl t-butyl ether (100:0) to (80:20)] to give 3-(4-t-butylphenyl)-cyclobutanone (3.80g, 95%).

Formic acid (1ml) was added to 3-(4-t-butylphenyl)-cyclobutanone (1.32g, 6.5mmol) and cis 2,6-dimethylmorpholine (0.75g, 6.5mmol) and the mixture stirred at 130°C for 7 hours. The mixture was cooled, ether added

and the mixture washed with water (x3), dried ($Na_2SO_4$) and evaporated in vacuo. Chromatography [$SiO_2$, methyl t-butyl ether-hexane(10:90) to (50:50)] gave Z 3-(4-t-butylphenyl)-1-[4-(cis 2,6-dimethylmorpholino)]cyclobutane (0.600g, 31%) (Compound No. 1 of Table I) and E 3-(4-t-butylphenyl)-1-[4-(cis 2,6-dimethylmorpholino)]-cyclobutane (0.234g, 12%) (Compound No. 2 of Table I).

## EXAMPLE 2

This Example illustrates the preparation of 3-(4-t-butylphenylmethyl)-1-[4-(cis 2,6-dimethylmorpholino)]-cyclobutane (Compound No. 4 of Table I).

4-t-Butylbromobenzene (24.5g, 115mmol) in dry ether (55ml) was added to a stirred mixture of magnesium turnings (3.05g, 125mmol) and a catalytic amount of iodine in dry ether (10ml) at such a rate so as to maintain reflux. After refluxing for a further 45 minutes allyl bromide (12.1g, 100mmol) was added at such a rate so as to maintain reflux. After a further 30 minutes copper (I) bromide (catalytic) was added. The mixture was poured onto ice (50g) and 2M hydrochloric acid (40ml) was added and the mixture extracted with ether. The extracts were dried ($MgSO_4$) and evaporated in vacuo. Chromatography ($SiO_2$, hexane) gave 3-(4-t-butylphenyl)prop-1-ene (13.77g, 79%).

Trichloroacetyl chloride (12.4ml, 111mmol) in dry ether (40ml) was added over 30 minutes to a mixture of zinc powder (9.8g, 150mmol) and 3-(4-t-butylphenyl)prop-1-ene (13.0g, 74.5mmol) in dry ether (250ml) placed in a sonic bath. When no 3-(4-t-butylphenyl)prop-1-ene remained (about 1 hour) the mixture was filtered (celite) and the filtrate washed with water, saturated sodium

bicarbonate solution and water, dried ($MgSO_4$) and evaporated *in vacuo*. Chromatography [$SiO_2$, hexane-ethyl acetate (95:5)] gave 3-(4-*t*-butylphenylmethyl)-2,2-dichlorocyclobutanone (5.44g, 26%).

3-(4-*t*-Butylphenylmethyl)-2,2-dichlorocyclobutanone (5.44g, 19mmol) and azobisisobutyronitrile (0.05g) in dry cyclohexane (40ml) were slowly added over 30 minutes to a stirred refluxing solution of tri-*n*-butyltin hydride (11ml, 41mmol) in dry cyclohexane (20ml). After 3 hours the mixture was cooled to room temperature and evaporated *in vacuo*. Chromatography [$SiO_2$, hexane-ethyl acetate (100:0) to (80:20)] gave 3-(4-*t*-butylphenylmethyl)cyclobutanone (0.91g, 20%) m.p. 49-51°C.

Formic acid (0.35ml) was added to a stirred mixture of 3-(4-*t*-butylphenylmethyl)cyclobutanone (0.50g, 2.3mmol) and *cis* 2,6-dimethylmorpholine (0.53g, 4.6mmol). The mixture was heated at 140°C for 7 hours and then cooled to room temperature. Ether was added and the mixture washed with saturated sodium bicarbonate solution and water (x3), dried ($MgSO_4$) and evaporated *in vacuo*. Chromatography [$SiO_2$, hexane-ethyl acetate (90:10) to (50:50)] gave 3-(4-*t*-butylphenylmethyl)-1-[4-(*cis* 2,6-dimethylmorpholino)]cyclobutane (0.18g, 25%) (Compound No. 4 of Table I).

EXAMPLE 3

This Example illustrates the preparation of *Z* and *E* 3-(4-*t*-butylphenyl)-1-[4-(*cis* 2,6-dimethylmorpholino)]-cyclopentane (Compound Nos. 7a and 7b of Table I).

*n*-Butyllithium (25.0ml of a 1.6*M* solution in hexane, 40mmol) was added dropwise to a stirred solution of 4-bromo-*t*-butylbenzene (8.52g, 40mmol) in dry ether (80ml) at 0°C under nitrogen. After 30 minutes at 0°C 3-ethoxy-cyclopent-2-enone (2.40ml, 20mmol) in dry ether (10ml) was added. After 15 minutes at 0°C and 15 minutes at room

temperature the mixture was poured onto 2M hydrochloric acid (100ml). The organic layer was separated and the aqueous layer extracted with ether (x2). The combined organic fractions were washed with water, saturated aqueous sodium bicarbonate solution, water and brine, dried ($MgSO_4$) and evaporated *in vacuo*. Chromatography [$SiO_2$, hexane-ethyl acetate (80:20) to (40:60)] gave 3-(4-*t*-butylphenyl)cyclopent-2-enone (3.04g, 71%).

3-(4-*t*-Butylphenyl)cyclopent-2-enone (1.0g, 4.67mmol) and 10% palladium on charcoal (~0.2g) were stirred in methanol (11ml) and triethylamine (2.2ml) under a hydrogen atmosphere (40psi) for 5 hours when hydrogen uptake was complete. The mixture was filtered (celite) and the filtrate evaporated *in vacuo* to give 3-(4-*t*-butylphenyl)-cyclopentanone (0.966g, 96%).

Hydrogen chloride in methanol (3.6ml of a 2.5M solution, 9.0mmol) was added dropwise to a stirred mixture of 3-(4-*t*-butylphenyl)cyclopentanone (0.966g, 4.5mmol), tetrabutylammonium cyanoborohydride (2.52g, 8.9mmol), *cis* 2,6-dimethylmorpholine (3.10g, 27mmol) and 4A molecular sieves ( 1g) in dry dichloromethane (50ml) under nitrogen. After 48 hours the mixture was filtered and the filtrate washed with 2M sodium hydroxide solution (x2), water (x4), dried ($MgSO_4$), and evaporated *in vacuo*. Chromatography [$SiO_2$, hexane-ethyl acetate (50:50)] gave *Z* 3-(4-*t*-butylphenyl)-1-[4-*cis* 2,6-dimethyl-morpholino)]cyclopentane (0.751g, 53%) (Compound No. 7a of Table I) and *E* 3-(4-*t*-butylphenyl)-1-[4-(*cis* 2,6-dimethylmorpholino)]cyclopentane (0.340g, 24%) (Compound No. 7b of Table I).

## EXAMPLE 4

This Example illustrates the preparation of *Z* and *E* 3-(4-*i*-propylphenyl)-1-[4-(*cis* 2,6-dimethylmorpholino)]-cyclopentane (Compound Nos. 8a and 8b of Table I).

0259977

Lithium (0.28g, 40mmol) was added to a mixture of 4-*i*-propylbromo-benzene (4.04g, 20.3mmol) and zinc bromide (2.25g, 10mmol) in dry ether (60ml) under nitrogen. The mixture was sonocated under reflux until virtually all the lithium had disappeared (about 2 hours). 2-Cyclopentenone (0.41g, 5mmol) and nickel acetylacetonate (30mg, 0.1mmol) in dry ether (5ml) were added. After 20 hours the mixture was poured onto saturated aqueous ammonium chloride solution. The mixture was extracted with ether (x2) and the combined extracts dried ($MgSO_4$) and evaporated *in vacuo*. Chromatography [$SiO_2$, hexane-methyl *t*-butyl ether (95:5) to (90:10)] gave 3-(4-*i*-propylphenyl)cyclopentanone (0.34g, 34%).

Hydrogen chloride in methanol (1.25ml of a 2*M* solution, 2.5mmol) was added to a stirred mixture of 3-(4-*i*-propylphenyl)cyclopentanone (0.228g, 1.1mmol), sodium cyanoborohydride (0.05g, 0.8mmol), aliquat 336 (0.325g, 0.8mmol), *cis* 2,6-dimethylmorpholine (0.80g, 7mmol) and 4A molecular sieves (~1g) in dry dichloromethane (13ml) under nitrogen. After 48 hours the mixture was filtered and the filtrate washed with 2*M* sodium hydroxide solution (x2) and water, dried ($MgSO_4$) and evaporated *in vacuo*. Chromatography [$SiO_2$, *t*-butyl methyl ether-hexane (20:80) to (50:50)] gave *Z* 3-(4-*i*-propylphenyl)-1-[*cis* 2,6-dimethylmorpholino)]cyclopentane (0.070g, 21%) (Compound No. 8a of Table I) and *E* 3-(4-*i*-propylphenyl)-1-[*cis* 2,6-dimethylmorpholino)]cyclopentane (0.022g, 7%) (Compound No. 8b of Table I).

## EXAMPLE 5

This Example illustrates the preparation of *E* and *Z* 3-(4-*t*-butylphenyl)-1-[4-(*cis* 2,6-dimethylmorpholino)]-cyclohexane (Compound Nos. 10 and 11 of Table I).

*n*-Butyllithium (7.2ml of a 1.6*M* solution in hexane, 10mmol) was added to a stirred solution of 4-bromo-*t*-butylbenzene (2.13g, 10mmol) in dry ether (20ml) at 0°C under nitrogen. After 2 hours at room temperature the mixture was cooled to 0°C and added dropwise to a stirred suspension of copper (I) bromide dimethylsulphide complex (1.24g, 6mmol) in dry ether (20ml) at -15°C over 5 minutes. After stirring at -10 to -15°C for 20 minutes 2-cyclohexenone (0.192g, 2mmol) in dry ether (10ml) was added dropwise. After 2 hours at -10 to -15°C methanol was added dropwise and the mixture poured onto aqueous ammonium chloride and ammonium hydroxide solution. The mixture was extracted with ether and the extracts washed with 3% aqueous ammonia solution (x3), water and brine, dried ($MgSO_4$) and evaporated in vacuo. Chromatography [$SiO_2$, ethyl acetate-hexane (0:100) to (20:80)] gave the 3-(4-t-butylphenyl)cyclohexanone (0.49g, 82% pure, 87%) which was used without further purification.

cis-2,6-Dimethylmorpholine (0.35g, 3mmol) was added dropwise to 3-(4-t-butylphenyl)cyclohexanone (0.49g, 82% pure, 1.75mol). After stirring for 1 hour formic acid (0.2ml) was added dropwise and after 20 hours at room temperature the mixture was heated at 140°C for a total of 10 hours. The mixture was partitioned between 2M hydrochloric acid and ether and the ether further extracted with 2M hydrochloric acid (x2). The combined acidic extracts were brought to approximately pH9 and extracted with ether (x3). The combined ether extracts were washed with water and brine, dried ($MgSO_4$) and evaporated in vacuo. Chromatography [$SiO_2$, ethyl acetate-hexane (0:100) to (20: 80)] gave E 3-(4-t-butylphenyl)-1-[4-(cis 2,6-dimethylmorpholino)]cyclohexane (0.074g, 13%) (Compound No. 10 of Table I) and Z 3-(4-t-butylphenyl)-1-[4-(cis 2,6-dimethyl-morpholino)]cyclohexane (0.102g, 18%) (Compound No. 11 of Table I).

EXAMPLE 6

This Example illustrates the preparation of Z and E 3-(4-trimethylsilylphenyl)-3-methyl-1-[4-(cis 2,6-dimethylmorpholino)]cyclobutane (Compound Nos. 37 and 38 of Table I).

Dimethyl sulphoxide (35ml) was added to hexane-washed sodium hydride (2.5g of a 50% suspension in oil, 50mmol) under argon. The mixture was stirred and heated at 70°C for 1 hour and then cooled to 0°C. Tetrahydrofuran (40ml) was added followed by methyltriphenylphosphonium bromide (18g, 50mmol) portionwise. After 30 minutes 4-bromoacetophenone (10g, 50mmol) was slowly added. After 24 hours the mixture was poured onto water and extracted with hexane. The combined extracts were washed with water, dried ($MgSO_4$) and evaporated in vacuo. Chromatography ($SiO_2$, hexane) gave 2-(4-bromophenyl)prop-1-ene (7.5g, 76%).

n-Butyllithium (24.3ml of a 1.6M solution in hexane, 39mmol) was added dropwise to a stirred solution of 2-(4-bromophenyl)prop-1-ene (7.50g, 38mmol) in dry tetrahydrofuran (52ml) under nitrogen at such a rate so as to keep the temperature below -60°C. After 1½ hours the mixture was warmed to -55°C and trimethylsilyl chloride (4.9ml, 38mmol) was added dropwise. After 30 minutes the mixture was warmed to room temperature and ethyl acetate (1ml) added. The mixture was poured onto water and the organic fraction separated, washed with water (x3) and brine, dried ($MgSO_4$) and evaporated in vacuo to give 2-(4-trimethylsilyl)prop-1-ene (6.5g, 90%).

Trifluoromethanesulphonic anhydride (0.9ml, 5.4mmol) in dry chloroform (37ml) was added over a period of 7 hours to a stirred solution of 2-(4-trimethylsilylphenyl)-prop-1-ene (2.80g, 14.7mmol), N-acetyl cis 2,6-dimethylmorpholine (0.75g, 4.8mmol) and lutidine (0.59g, 5.5mmol)

in dry chloroform (20ml) under reflux. The mixture was cooled to room temperature and tetrabutylammonium cyanoborohydride (2.02g, 7.2mmol) was added. After 16 hours the mixture was washed with sodium hydroxide solution, dried ($MgSO_4$) and evaporated in vacuo. Chromatography [$SiO_2$, hexane-ethyl acetate (100:0) to (50:50)] gave Z 3-(4-trimethylsilylphenyl)-3-methyl-1-[4-(cis 2,6-dimethylmorpholino)]cyclobutane (0.733g, 46%) (Compound No. 37 of Table I) and E 3-(4-trimethylsilyl-phenyl)-3-methyl-1-[4-(cis 2,6-dimethylmorpholino)]cyclobutane (0.348g, 22%) (Compound No. 38 of Table I).

## EXAMPLE 7

This Example illustrates the preparation of Z and E 3-(4-chlorophenyl)-1-[4-(cis 2,6-dimethylmorpholino)]-cyclopentane (Compound Nos. 39 and 40 of Table I).

n-Butyllithium (26.0ml of a 1.55M solution, 40.3mmol) was added dropwise to a stirred solution of 4-chlorobromo-benzene (7.66g, 40mmol) in dry ether (60ml) at 0°C under nitrogen. After 1 hour at room temperature the mixture was added to zinc bromide (4.5g, 20mmol) in dry ether (20ml) and the mixture sonocated for about 30 minutes until all the zinc bromide had dissolved. 2-Cyclopentenone (0.82g, 10mmol) and nickel acetyl-acetonate (0.060g, 0.2mmol) in dry ether (10ml) were then added at room temperature. After 1 hour the mixture was poured into saturated aqueous ammonium chloride solution and the mixture extracted with ether. The combined extracts were dried ($MgSO_4$) and evaporated in vacuo. Chromatography [$SiO_2$, hexane-ethyl acetate (80:20)] gave 3-(4-chlorophenyl)cyclopentanone (1.03g, 53%).

Hydrogen chloride in methanol (1.3ml of a 2.5M solution, 3.25mmol) was added to a stirred mixture of 3-(4-chlorophenyl)cyclopentanone (0.32g, 1.65mmol), tetrabutylammonium cyanoborohydride (0.88g, 3.1mmol), cis 2,6-dimethylmorpholine (1.14g 9.9mmol) and 4A molecular sieves ( 1g) in dry dichloromethane (20ml) under nitrogen. After 48 hours the mixture was filtered and the filtrate washed with 2M sodium hydroxide solution (x2) and water (x2), dried ($MgSO_4$) and evaporated in vacuo. Chromatography [$SiO_2$, hexane-t-butyl methyl ether (20:80)] gave Z 3-(4-chlorophenyl)-1-[4-(cis 2,6-dimethylmorpholino)]cyclopentane (74mg, 15%) (Compound No. 39 of Table I) and E 3-(4-chlorophenyl)-1-[4-(cis 2,6-dimethylmorpholino)]cyclopentane (41mg, 9%) (Compound No. 40 of Table I).

## EXAMPLE 8

This Example illustrates the preparation of Z 2-(4-t-butylphenyl)-1-[4-(cis 2,6-dimethylmorpholino)]cyclopentane (Compound No. 48 of Table I).

4-t-Butylbromobenzene (4.50g, 21.1mmol) in dry ether (10ml) was added to a stirred mixture of magnesium (0.56g, 23.2mmol) in dry ether (5ml) under nitrogen. Once reaction had been initiated the addition was performed at such a rate so as to maintain reflux. After the addition was complete the mixture was stirred under reflux for 40 minutes and then cooled to room temperature. The mixture was then added dropwise to 2-chlorocyclopentanone (2.5g, 21.1mmol) in dry toluene (10ml) at 0°C under nitrogen. The mixture was then allowed to warm to room temperature and then heated at 80°C for 1½ hours. The mixture was then cooled to room temperature and poured onto dilute aqueous ammonium chloride solution and the mixture extracted with ether. The combined extracts were dried ($MgSO_4$) and evaporated in vacuo. Chromatography [$SiO_2$, hexane-ethyl acetate (80:20)] gave 2-(4-t-butylphenyl)-cyclopentanone (1.78g, 39%).

Hydrogen chloride in methanol (1.5ml of a 2.5M solution, 3.75mmol) was added to a stirred mixture of 2-(4-t-butylphenyl)cyclopentanone (0.40g, 1.85mmol), sodium cyanoborohydride (0.23g, 3.7mmol), aliquat 336 (1.48g, 3.7mmol), cis 2,6-dimethylmorpholine (1.28g, 11.1mmol) and 4A molecular sieves (~1g) in dry dichloromethane (30ml). After 48 hours the mixture was filtered (celite) and the filtrate washed with 2M sodium hydroxide solution (x2) and water, dried ($MgSO_4$) and evaporated in vacuo. Chromatography [$SiO_2$, hexane-t-butyl methyl ether (50:50)] gave Z 2-(4-t-butylphenyl)-1-[4-(cis 2,6-dimethyl-morpholino)]cyclopentane (50mg, 11%) (Compound No. 48 of Table I).

## EXAMPLE 9

This Example illustrates the preparation of Z and E 3-(4-t-butylphenyl)-1-[4-(cis 2,6-dimethylmorphlino)]-3-methylcyclopentane (Compound Nos. 53 and 54 of Table I).

Lithium wire (0.56g, 80.6mmol) was added to a mixture of 4-t-butylbromobenzene (8.52g, 40mmol) and zinc bromide (4.50g, 20mmol) in dry ether (100ml) under nitrogen. The mixture was sonocated under reflux until all the lithium had disappeared (about 2½ hours). The mixture was cooled to room temperature and 3-methyl-2-cyclopentenone (0.66ml, 9.7mmol) and nickel acetylacetonate (0.06g, 0.2mmol) in dry ether (10ml) were added. After 18 hours the mixture was poured onto saturated aqueous ammonium chloride solution and the mixture extracted with ether (x3). The combined extracts were dried ($MgSO_4$) and evaporated in vacuo. Chromatography [$SiO_2$, hexane-ethyl acetate (80:20)] gave 3-(4-t-butylphenyl)-3-methylcyclopentanone (0.58g, 63%).

Hydrogen chloride in methanol (1.92ml of a 2.6M solution, 5.0mmol) was added to a stirred solution of 3-(4-t-butylphenyl)-3-methylcyclopentanone (0.58g, 2.52mmol), tetrabutylammonium cyanoborohydride (1.42g, 5.0mmol), cis 2,6-dimethylmorpholine (1.48g, 12.9mmol) and 4A molecular sieves (~1g) in dry dichloromethane (30ml). After 48 hours the mixture was filtered and the filtrate washed with 2M sodium hydroxide solution (x2) and water, dried ($MgSO_4$) and evaporated in vacuo. Chromatography [$SiO_2$, hexane-ethyl acetate (70:30)] gave Z 3-(4-t-butylphenyl)-1-[4-(cis 2,6-dimethylmorpholino)]-3-methylcyclopentane (0.356g, 43%) (Compound No. 53 of Table I) and E 3-(4-t-butylphenyl)-1-[4-(cis 2,6-dimethylmorpholino)]-3-methyl-cyclopentane (0.348g, 42%) (Compound No. 54 of Table I).

EXAMPLE 10

This Example illustrates the preparation of 3-(4-i-propylphenyl)-1-methyl-1-[4-(cis 2,6-dimethylmorpholino)]-cyclobutane (Compound No. 61 of Table I).

Trifluoromethanesulphonic anhydride (0.97ml, 5.8mol) in dry chloroform (32ml) was added over a period of 7 hours to a stirred solution of 4-i-propylstyrene (2.3g, 16mmol), N-acetyl cis 2,6-dimethylmorpholine (0.80g, 5mmol) and lutidine (0.63g, 6mmol in dry chloroform (20ml) under reflux. The mixture was cooled to -30°C and methylmagnesium bromide (6ml of a 3M solution in ether, 18mmol) added slowly. The mixture was warmed to room temperature and after 30 minutes methanol (25ml) was added. The mixture was poured onto water and extracted wtih ether. The extracts were washed with 2M sodium hydroxide solution (x3) and water (x5), dried ($MgSO_4$) and evaporated in vacuo. The resultant oil was dissolved in ether and extracted with 2M hydrochloric acid. The extracts were basified (2M sodium hydroxide solution) and extracted with ether. The extracts were dried ($MgSO_4$) and evaporated in vacuo to give 3-(4-i-propylphenyl)-1-methyl-1-[4-(cis 2,6-

dimethylmorpholino)]cyclobutane (0.29g, 19%) as a 2:1 mixture of Z and E isomers (Compound No. 61 of Table I).

EXAMPLE 11

This Example illustrates the preparation of 3-(4-chlorophenylmethyl)-1-[4-(cis 2,6-dimethylmorpholino)]cyclopentane (Compound No. 71 of Table I).

4-Chlorobenzaldehyde (14.05g, 100mmol) in dry dimethylformamide (50ml) was added dropwise (over a period of 10 minutes) to a stirred mixture of sodium cyanide (2.45g, 50mmol) in dry dimethylformamide (50ml) at 35°C under nitrogen. After 5 minutes 2-cyclopentenone (6.15g, 75mmol) in dry dimethylformamide (100ml) was added over a period of 30 minutes, maintaining the temperature at 35°C. After 2 hours the mixture was poured onto water (400ml), brine was added and the mixture extracted with chloroform. The combined extracts were washed with water, dried ($MgSO_4$) and evaporated in vacuo. Chromatography [$SiO_2$, hexane-ethyl acetate (80:20)] gave 3-(4-chlorophenylcarbonyl)cyclopentanone (6.13g, 37%).

Hydrogen chloride in methanol (14.0ml of a 2.6M solution, 37.3mmol) was added to a stirred mixture of 3-(4-chlorophenylcarbonyl)cyclopentanone (4.15g, 18.6mmol), tetrabutylammonium cyanoborohydride (7.98g, 22.8mmol), cis 2,6-dimethylmorpholine (12.87g, 112mmol) and 4A molecular sieves (~1g) in dry dichloromethane (200ml). After 48 hours the mixture was filtered and the filtrate washed with 2M sodium hydroxide solution (x2) and water, dried ($MgSO_4$) and evaporated in vacuo. The resultant oil was dissolved in ether and the mixture extracted with 2M hydrochloric acid. The combined extracts were washed with ether, made basic with 2M sodium hydroxide solution and extracted with dichloromethane. The extracts were washed with sodium bicarbonate solution, dried ($MgSO_4$) and evaporated in vacuo to give 3-(4-chlorophenylcarbonyl)-1-[4-(cis 2,6-dimethylmorpholino)]cyclopentane (1.64g, 27%).

0259977

Triethylsilane (0.62ml, 3.9mmol), was added to a stirred mixture of 3-(4-chlorophenylcarbonyl)-1-[4-(cis-2,6-dimethylmorpholino)]cyclopentane (0.50g, 1.56mmol) in trifluoroacetic acid (1.20ml, 15.6mmol) under nitrogen. After 48 hours triethylsilane (0.62ml, 3.9mmol) and trifluoroacetic acid (1.20ml, 15.6mmol) were added. After 48 hours ether was added and the mixture washed with water, saturated aqueous sodium bicarbonate, water and brine, dried ($MgSO_4$), and evaporated in vacuo. Chromatography [$SiO_2$, hexane-ethyl acetate (70:30) to (50:50)] gave 3-(4-chlorophenylmethyl)-1-[4-(cis 2,6-dimethylmorpholino)]cyclopentane (0.089g, 19%) (Compound No. 71 of Table I).

EXAMPLE 12

This Example illustrates the preparation of 3-(4-t-butylphenyl)-1-[4-(cis 2,6-dimethylmorpholino)]-5-methyl-cyclopentane (Compound Nos. 72 and 73 of Table I).

n-Butyllithium (9.90ml of a 1.6M solution in hexane, 15.8mmol) was added dropwise to stirred diisopropylamine (2.20ml, 15.8mmol) at 0°C under nitrogen. After 20 minutes the mixture was cooled to -78°C and 3-(4-t-butylphenyl)cyclopent-2-enone (3.0g, 14mmol) in dry tetrahydrofuran (20ml) added. The mixture was allowed to warm to room temperature and after 30 minutes methyl iodide (1.75ml, 28mmol) was added. After 30 minutes the mixture was poured onto saturated aqueous ammonium chloride solution and the mixture extracted with ether. The combined extracts were washed with water and brine, dried ($MgSO_4$) and evaporated in vacuo. Chromatography [$SiO_2$, hexane-ethyl acetate (80:20)] gave 3-(4-t-butylphenyl)-5-methylcyclopent-2-enone (2.01g, 63%).

A solution of 3-(4-t-butylphenyl)-5-methylcyclopent-2-enone (2.00g, 8.8mmol), in methanol (15ml) and

triethylamine (4ml) was hydrogenated over 5% palladium on charcoal ( 0.1g) under 40 psi of hydrogen. After 8 hours when hydrogen uptake was complete the mixture was filtered and the filtrate evaporated in vacuo to give 3-(4-t-butylphenyl)-5-methylcyclopentanone (2.30g, 100%).

Hydrogen chloride in methanol (7.08ml of a 2.5M solution, 17.7mmol) was added dropwise to a stirred mixture of 3-(4-t-butylphenyl)-5-methylcyclopentanone (2.03g, 8.8mmol), tetrabutylammonium cyanoborohydride (5.02g, 17.7mmol), cis 2,6-dimethylmorpholine (6.07g, 52.9mmol) and 4A molecular sieves ( 1g) in dry dichloromethane (40ml) under nitrogen. After 48 hours the mixture was filtered and the filtrate washed with 2M sodium hydroxide solution. The aqueous extracts were extracted with ether and the combined organic extracts washed with water (x6) and brine, dried ($MgSO_4$) and evaporated in vacuo. Chromatography [$SiO_2$, hexane-ethyl acetate (70:30)] gave 1RS, 3RS, 5SR 3-(4-t-butylphenyl)-1-[4-(cis 2,6-dimethylmorpholino)]-5-methylcyclopentane (0.477g, 17%) (Compound No. 72 of Table I) and a 3:2 mixture of 1RS, 3SR, 5RS and 1RS, 3RS, 5RS 3-(4-t-butylphenyl)-1-[4-(cis 2,6-dimethylmorpholino)]-5-methylcyclopentane (0.780g, 27%) (Compound No. 73 of Table I).

## EXAMPLE 13

An emulsifiable concentrate was made up by mixing the ingredients, and stirring the mixture until all the constituents were dissolved.

| | |
|---|---|
| Compound No. 1 of Table I | 10% |
| Ethylene dichloride | 40% |
| Calcium dodecylbenzenesulphate | 5% |
| "Lubrol" L | 10% |
| "Aromasol" H | 35% |

EXAMPLE 14

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound No. 1 of Table I | 50% |
| "Dispersol" T | 25% |
| "Lubrol" APN5 | 1.5% |
| Sodium acetate | 23.5% |

EXAMPLE 15

The ingredients were all ground together to produce a powder formulation readily dispersible in liquids.

| | |
|---|---|
| Compound No. 1 of Table I | 45% |
| "Dispersol" T | 5% |
| "Lissapol" NX | 0.5% |
| "Cellofas" B600 | 2% |
| Sodium acetate | 47.5% |

EXAMPLE 16

The active ingredient was dissolved in a solvent and the resultant liquid was sprayed on to the granules of China clay. The solvent was then allowed to evaporate to produce a granular composition.

| | |
|---|---|
| Compound No. 1 of Table I | 5% |
| China clay granules | 95% |

EXAMPLE 17

A composition suitable for use as a seed dressing was prepared by mixing the three ingredients.

| | |
|---|---|
| Compound No. 1 of Table I | 50% |
| Mineral oil | 2% |
| China clay | 48% |

EXAMPLE 18

A dusting powder was prepared by mixing the active ingredient with talc.

| | |
|---|---|
| Compound No. 1 of Table I | 5% |
| Talc | 95% |

EXAMPLE 19

A Col formulation was prepared by ball-milling the constituents set out below and then forming an aqueous suspension of the ground mixture with water.

| | |
|---|---|
| Compound No. 1 of Table I | 40% |
| "Dispersol" T | 10% |
| "Lubrol" APN5 | 1% |
| Water | |

EXAMPLE 20

A dispersible powder formulation was made by mixing together the ingredients set out below and then grinding the mixture until all were thoroughly mixed.

| | |
|---|---|
| Compound No. 1 of Table I | 25% |
| "Aerosol" OT/B | 2% |

| | |
|---|---|
| "Dispersol" A.C. | 5% |
| China clay | 28% |
| Silica | 40% |

EXAMPLE 21

This Example illustrates the preparation of a dispersible powder formulation. The ingredients were mixed and the mixture then ground in a comminution mill.

| | |
|---|---|
| Compound No. 1 of Table I | 25% |
| "Perminal" BX | 1% |
| "Dispersol" T | 5% |
| Polyvinylpyrrolidone | 10% |
| Silica | 25% |
| China clay | 34% |

EXAMPLE 22

The ingredients set out below were formulated into a dispersible powder by mixing then grinding the ingredients.

| | |
|---|---|
| Compound No. 1 of Table I | 25% |
| "Aerosol" OT/B | 2% |
| "Dispersol" A | 5% |
| China clay | 68% |

In Examples 13 to 22 the proportions of the ingredients given are by weight.

The remaining compounds set out in Table I were similarly formulated as specifically described in Examples 13 to 22.

There now follows an explanation of the compositions or substances represented by the various Trade Marks mentioned above.

| | |
|---|---|
| LUBROL L : | a condensate of nonyl phenol 1 mole) with ethylene oxide (13 moles) |
| AROMASOL H : | a solvent mixture of alkylbenzenes |
| DISPERSOL T & AC : | a mixture of sodium sulphate and a condensate of formaldehyde with sodium naphthalene sulphonate |
| LUBROL APN5 : | a condensate of nonyl phenol (1 mole) with naphthalene oxide (5.5 moles) |
| CELLOFAS B600 : | a sodium carboxymethyl cellulose thickener |
| LISSAPOL NX : | a condensate of nonyl phenol (1 mole) with ethylene oxide (8 moles) |
| AEROSOL OT/B : | dioctyl sodium sulphosuccinate |
| PERMINAL BX : | a sodium alkyl naphthalene sulphonate |

EXAMPLE 23

The compounds were tested against a variety of foliar fungal diseases of plants. The technique employed was as follows.

The plants were grown in John Innes Potting Compost (No 1 or 2) in 4 cm diameter minipots. The test compounds were formulated either by bead milling with aqueous Dispersol T or as a solution in acetone or acetone/ethanol

which was diluted to the required concentration immediately before use. For the foliage diseases, the formulations (100 ppm active ingredient) were sprayed on to the foliage and applied to the roots of the plants in the soil. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the disease. An exception was the test on Erysiphe graminis in which the plants were inoculated 24 hours before treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in Table II.

TABLE II

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 1 | 1 | 4 | 0 | 4 |
| 2 | 1 | 4 | 3 | 4 |
| 3 | 0 | 4 | 0 | 0 |
| 4 | 3 | 4 | 4 | 4 |
| 6a | 0 | 4 | 4 | 4 |
| 6b | 0 | 4 | 4 | 3 |
| 7a | 0 | 4 | 4 | 4 |
| 7b | 3 | 4 | 4 | 4 |
| 8a | 0 | 4 | 2 | 4 |
| 8b | 0 | 4 | 0 | 0 |
| 9 | 3 | 4 | 0 | 3 |
| 10 | 0 | 4 | 0 | 0 |
| 11 | 0 | 4 | 0 | 0 |
| 14 | 0 | 4 | 4 | 3 |
| 15 | - | - | - | - |
| 16 | 0 | 4 | 4 | 4 |
| 17 | 0 | 4 | 0 | 0 |
| 18 | 0 | 4 | 4 | 4 |
| 19 | 4 | 4 | 4 | 4 |
| 20 | 4 | 4 | 0 | 4 |
| 21 | 0* | 2* | 0* | 0* |
| 22 | 0 | 4 | 0 | 1 |
| 23 | 0 | 4 | 0 | 0 |
| 24 | 0 | 4 | 0 | 0 |
| 25 | 0 | 1 | 0 | 0 |

TABLE II (CONT/D)

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 26 | 0 | 2 | 0 | 0 |
| 27 | 0 | 4 | 0 | 0 |
| 29 | 0 | 4 | 0 | 0 |
| 30 | 0 | 3 | 0 | 0 |
| 31 | 0* | 4* | 0* | 0* |
| 32 | 0* | 3* | 0* | 0* |
| 33 | 0 | 3 | 0 | 0 |
| 34 | 0 | 4 | 0 | 0 |
| 35 | 0 | 3 | 1 | 0 |
| 36 | 3 | 4 | 0 | 0 |
| 37 | 0 | 4 | 3 | 3 |
| 38 | 0 | 4 | 0 | 0 |
| 39 | 0 | 4 | 0 | 0 |
| 40 | 0 | 4 | 0 | 0 |
| 41 | 0 | 4 | 0 | 0 |
| 42 | 0 | 4 | 0 | 0 |
| 43 | 0 | 4 | 4 | 4 |
| 44 | 0* | 4* | 0* | 4* |
| 45 | 0 | 4 | 4 | 4 |
| 46 | 0 | 4 | 4 | 0 |
| 47 | 0 | 4 | 0 | 0 |
| 48 | - | 2** | 3 | 0 |
| 49 | 0 | 4 | 4 | 3 |
| 50 | 0 | 4 | 4 | 0 |
| 51 | - | 4** | - | - |
| 52 | 2 | 4 | 4 | 4 |
| 53 | 0 | 4 | 3 | 4 |
| 54 | 1 | 4 | 4 | 4 |
| 55 | 0 | 4 | 0 | 0 |

0259977

TABLE II (CONT/D)

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 57 | 0 | 4 | 4 | 4 |
| 58 | 0* | 4* | 4* | 0* |
| 59 | 0* | 4* | 4* | 1* |
| 60 | 0* | 4* | 4* | 3* |
| 61 | 1 | 4 | 2 | 3 |
| 62 | 0 | 1 | 3 | 4 |
| 63 | 0 | 4 | 4 | 3 |
| 64 | 0 | 4 | 4 | 3 |
| 65 | - | - | - | - |
| 66 | 0 | 0 | 4 | 4 |
| 67 | 0 | 4 | 0 | 4 |
| 68 | 0 | 4 | 4 | 2 |
| 69 | - | - | - | - |
| 70 | 2 | 4 | 0 | 3 |
| 71 | 0* | 4* | 0* | 3* |
| 72 | 0 | 4 | 4 | 4 |
| 73 | 0 | 4 | 4 | 0 |
| 74 | 0 | 4 | 2 | 4 |
| 76 | - | - | - | - |
| 77 | - | - | - | - |

* = 25ppm Foliar spray
** = 100ppm Foliar spray
\- = Results not yet available

PP 34022
HGHA/dlc
4 Aug 87

1. A compound having the general formula (I):

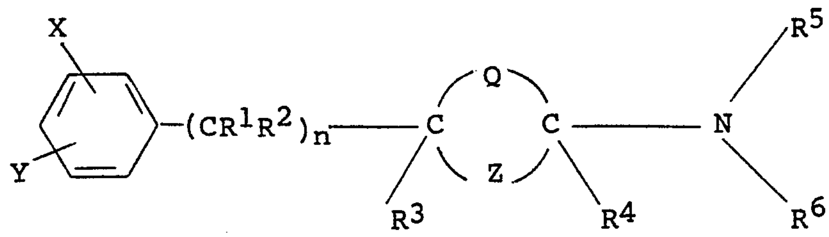

(I)

and stereoisomers thereof, wherein $R^1$, $R^2$ and $R^3$ each represents a hydrogen atom, a halogen atom, or an alkyl group containing from 1 to 4 carbon atoms, $R^4$ represents a hydrogen atom or an alkyl group containing from 1 to 4 carbon atoms, $R^5$ and $R^6$ each represents an alkyl group containing from 1 to 4 carbon atoms or $R^5$ and $R^6$ together with the adjacent nitrogen atom form a heterocyclic ring which may optionally contain an additional heteroatom, X and Y each represent a hydrogen atom, a halogen atom, or an alkyl, cycloalkyl, cycloalkylalkyl, alkenyl, alkynyl, aryl, aralkyl, alkoxy or aryloxy group,or a

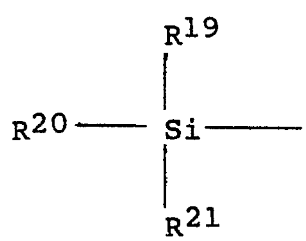

group wherein $R^{19}$, $R^{20}$ and $R^{21}$ can be an alkyl, alkenyl, alkynyl, cycloalkyl, cycloalkylalkyl, aryl or aralkyl group, n has the value 0 or 1 and Q is a

bridging group having one of the following values $Q_1$, $Q_2$, $Q_3$ and $Q_4$ :

$Q_1$=

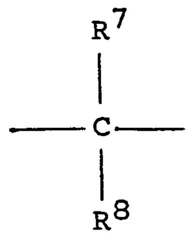

$Q_2$=

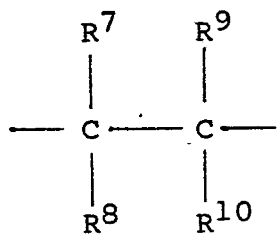

$Q_3$=

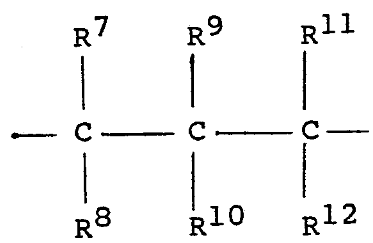

$Q_4$=

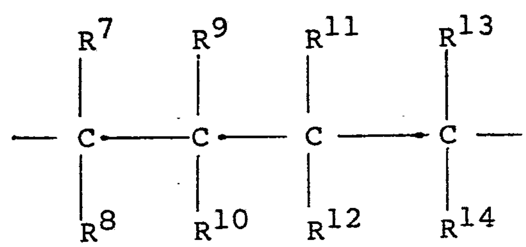

wherein $R^7$ to $R^{14}$ each independently represents a hydrogen atom, a halogen atom, a hydroxyl group or an alkyl group containing from 1 to 4 carbon atoms; Z is a bridging group which is a covalent linkage, denoted by $Z_o$, or has one of the following values $Z_1$ and $Z_2$:

$Z_1$=

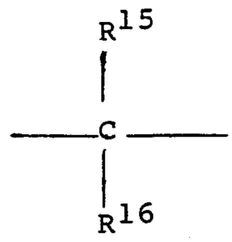

$Z_2$=

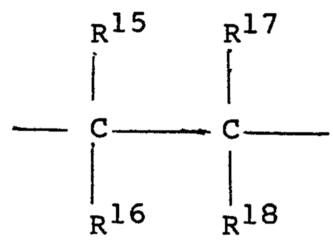

wherein $R^{15}$ to $R^{18}$ each independently has the same significance as $R^7$ to $R^{14}$ above; provided that

(i) when Z has the value $Z_o$, Q does not have the value $Q_1$;

(ii) the total number of carbon atoms forming part of the ring substance in formula (I) and contributed by the groups Q and Z does not exceed 4;

and acid addition salts and N-oxides thereof.

2. A compound as claimed in claim 1 wherein X and Y are a silyl group or are phenyl, benzyl, phenoxy or benzyloxy optionally substituted with one or more of: halogen (e.g. fluorine, chlorine or bromine), $C_{1-6}$ alkyl [e.g. methyl, ethyl, propyl (n- or iso-propyl) and butyl (n-, sec-, iso or tert-butyl)], $C_{1-6}$ alkoxy (e.g. methoxy, ethoxy, propoxy and butoxy), halo-$C_{1-6}$-alkyl (e.g. trifluoromethyl), halo-$C_{1-6}$-alkoxy (e.g. trifluoromethoxy), nitro, phenyl and phenoxy.

3. A compound as claimed in claim 1 or claim 2 wherein the X and Y substituents are at the 2-, 3- or 4-positions of the ring, particularly at the 3- or 4-positions and especially at the 4-position.

4. A compound as claimed in any of claims 1 to 3 wherein $R^5$ and $R^6$, together with the adjacent carbon atom, represent a piperidine, morpholine, thiomorpholine, pyrollidine or piperazine ring any of which may be optionally subsituted with one or more $C_{1-4}$ alkyl groups, phenyl or hydroxy $C_{1-4}$ alkyl.

5. A compound as claimed in any of claims 1 to 4 wherein X and Y are $C_{1-6}$ alkyl, $C_{3-6}$ cycloalkyl, $C_{4-9}$ cycloalkylalkyl, $C_{2-6}$ alkenyl, $C_{2-6}$ alkynyl.

6. A compound as claimed in any of claims 1 to 4 wherein Y is hydrogen and X is at the 3- or 4-position of the phenyl ring and is t-butyl, i-propyl or trimethylsilyl; n is 0 or 1; $R^1$, $R^2$, $R^3$ and $R^4$ are hydrogen, Q and Z are $-CH_2-$, $-(CH_2)_2-$, $-(CH_2)_3-$ or $CH.CH_3$; and $NR^5R^6$ is 2,6-dimethylmorpholine or piperidine.

7. Compounds as claimed in any of the preceding claims which are 1,3- cyclobutanes or 1,3-cyclopentanes, or 1,3- or 1,4-cyclohexanes.

8. The compound of formula:

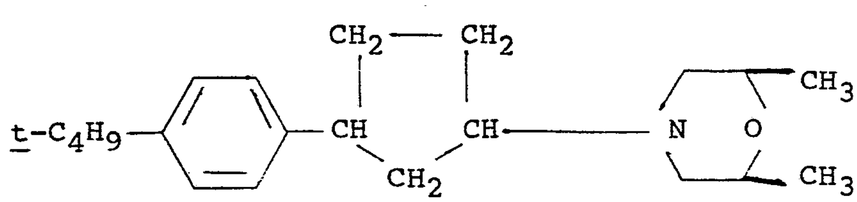

9. A process for preparing a compound as claimed in claim 1 and having the general formula (I):

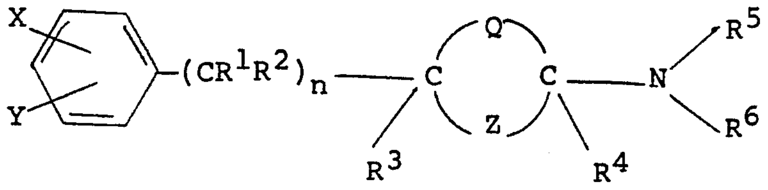

(I)

wherein $R^1$ to $R^3$, $R^5$, $R^6$, Q, X, Y, Z and n are as previously defined and $R^4$ is a hydrogen atom, which comprises the reductive amination of a compound of general formula (II):

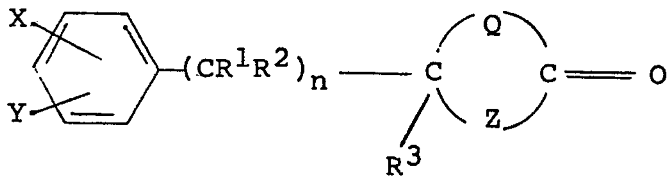

(II)

wherein X, Y, $R^1$, $R^2$, $R^3$, Q, X, Y, Z and n are as defined in claim 1 with an amine of general formula (III):

$$HNR^5R^6 \qquad (III)$$

wherein $R^5$ and $R^6$ are as defined in claim 1.

10. A fungicidal composition comprising a compound of general formula (I) as claimed in any of claims 1-7 hereinbefore defined, or a salt, thereof; and, optionally, a carrier or diluent.

11. A method of combating a fungi, which comprises applying to a plant, or seed of a plant, or to the locus of the plant or seed, a compound, or a salt thereof, as claimed in any of claims 1-7 or a composition as claimed in claim 9.